(19)

Europäisches
Patentamt
European
Patent Office
Office européen
des brevets

(11) **EP 2 246 070 A2**

(12) **EUROPEAN PATENT APPLICATION**

(43) Date of publication:
**03.11.2010 Bulletin 2010/44**

(51) Int Cl.:
***A61K 51/10*** *(2006.01)*   ***A61P 35/00*** *(2006.01)*

(21) Application number: **10008347.6**

(22) Date of filing: **01.09.2004**

(84) Designated Contracting States:
**AT BE BG CH CY CZ DE DK EE ES FI FR GB GR
HU IE IT LI LU MC NL PL PT RO SE SI SK TR**
Designated Extension States:
**AL HR LT LV MK**

(30) Priority: **10.09.2003 EP 03255633
10.09.2003 US 501881 P**

(62) Document number(s) of the earlier application(s) in
accordance with Art. 76 EPC:
**04764695.5 / 1 663 320**

(71) Applicants:
 • **Philogen S.p.A.
  53100 Siena (IT)**
 • **Bayer Schering Pharma Aktiengesellschaft
  13353 Berlin (DE)**

(72) Inventors:
 • **Borsi, Laura
  16132 Genova (IT)**

 • **Carnemolla, Barbara
  16132 Genova (IT)**
 • **Balza, Enrica
  16132 Genova (IT)**
 • **Castellani, Patrizia
  16132 Genova (IT)**
 • **Zardi, Luciano
  16132 Genova (IT)**
 • **Friebe, Matthias
  13509 Berlin (DE)**
 • **Hilger, Christoph-Stephan
  13503 Berlin (DE)**

(74) Representative: **Walton, Seán Malcolm et al
Mewburn Ellis LLP
33 Gutter Lane
London
EC2V 8AS (GB)**

Remarks:
This application was filed on 11-08-2010 as a
divisional application to the application mentioned
under INID code 62.

(54) **Targeting of tumor vasculature using radiolabelled antibody l19 against fibronectin ed-b**

(57)   A specific binding member that binds human ED-B, wherein the specific binding member is labelled with an isotope selected from the group consisting of 76Br, 77Br, 123I, 124I, 131I and 211At and comprises an antigen-binding site that comprises an antibody VH domain and an antibody VL domain, wherein the antibody VH domain is selected from the group consisting of the L19 VH domain, and a VH domain comprising a VH CDR1, a VH CDR2 and a VH CDR3, wherein the VH CDR3 is the L19 VH CDR3 of SEQ ID NO.3, the VH CDR1 is optionally L19 VH CDR1 of SEQ ID NO.1, and the VH CDR2 is optionally L19 VH CDR2 of SEQ ID NO. 2; and wherein the antibody VL domain is optionally selected from the group consisting of the L19 VL domain, and a VL domain comprising a VL CDR1, a VL CDR2 and a VL CDR3, wherein the VL CDR3 is the L19 VL CDR3of SEQ ID NO.6, the VL CDR1 is optionally L19 VL CDR1 of SEQ ID NO.4, and the VL CDR2 is optionally L19 VL CDR2 of SEQ ID NO.5; the L19 VH domain and L19 VL domain sequences being disclosed in Pini et al. (1998) J. Biol.Chem.273: 21769-21776; wherein the specific binding member comprises a mini-immunoglobulin comprising said antibody VH domain and antibody VL domain fused to eS2-CH4 and dimerized or comprises a whole IgG1 antibody molecule; also methods and uses employing such a specific binding member.

EP 2 246 070 A2

SEQUENCE LISTING

<110> Philogen SRL

Schering AG

Borsi, Laura

Carnemolla, Barbara

Balza, Enrica

Castellani, Patrizia

Zardi, Luciano

Friebe, Matthias

Hilger, Christoph-Stephan

<120>  Selective targeting of tumor vasculature using radiolabelled
antibody molecules

<130>  SMWFP6177075

<140>  EP 03255633.4
<141>  2003-09-10

<140>  US 60/501,881
<141>  2003-09-10

<150>
<151>

<160>  13

<170>  PatentIn version 3.1

<210>  1

<211>  5

<212>  PRT

<213>  Homo sapiens

```
<400>  1

Ser Phe Ser Met Ser
1               5


<210>  2

<211>  17

<212>  PRT

<213>  Homo sapiens


<400>  2

Ser Ile Ser Gly Ser Ser Gly Thr Thr Tyr Tyr Ala Asp Ser Val Lys
1               5                   10                  15

Gly


<210>  3

<211>  7

<212>  PRT

<213>  Homo sapiens


<400>  3

Pro Phe Pro Tyr Phe Asp Tyr
1               5


<210>  4

<211>  12

<212>  PRT

<213>  Homo sapiens


<400>  4

Arg Ala Ser Gln Ser Val Ser Ser Ser Phe Leu Ala
1               5                   10
```

```
<210>  5

<211>  7

<212>  PRT

<213>  Homo sapiens


<400>  5

Tyr Ala Ser Ser Arg Ala Thr
1               5


<210>  6

<211>  10

<212>  PRT

<213>  Homo sapiens


<400>  6

Cys Gln Gln Thr Gly Arg Ile Pro Pro Thr
1               5                   10


<210>  7

<211>  4

<212>  PRT

<213>  Artificial sequence


<220>

<223>  Linker peptide

<400>  7

Gly Gly Ser Gly
1


<210>  8

<211>  35

<212>  DNA
```

<213> Artificial sequence

<220>

<223> Primer

<400> 8
gtgtgcactc ggaggtgcag ctgttggagt ctggg                         35

<210> 9

<211> 36

<212> DNA

<213> Artificial sequence

<220>

<223> Primer

<400> 9
gcctccggat ttgatttcca ccttggtccc ttggcc                        36

<210> 10

<211> 33

<212> DNA

<213> Artificial sequence

<220>

<223> Primer

<400> 10
ctcgtgcact cgcaggtgca gctgcaggag tca                           33

<210> 11

<211> 30

<212> DNA

<213> Artificial sequence

```
<220>

<223>   Primer

<400>   11
ctctccggac cgtttgatct cgcgcttggt                         30


<210>   12

<211>   33

<212>   DNA

<213>   Artificial sequence


<220>

<223>   Primer

<400>   12
tggtgtgcac tcggaaattg tgttgacgca gtc                     33


<210>   13

<211>   30

<212>   DNA

<213>   Artificial sequence


<220>

<223>   Primer

<400>   13
ctctcgtacg tttgatttcc accttggtcc                         30
```

**Description**

The present invention relates to targeting of.tumor vasculature using radiolabelled antibody molecules. In particular, the invention relates to use of antibody molecules that bind ED-B of fibronectin, and which are of demonstrated usefulness in tumor targeting. In different embodiments of the present invention, antibody molecules are employed in different molecular formats. In certain embodiments the antibody molecules comprise human IgG1. In other embodiments the antibody molecules are mini-immunoglobulins, such as are generated by fusing an scFv antibody molecule to the constant CH4 domain of a secretory IgE isoform that naturally contains a cysteine in its COOH terminal which forms a covalently linked dimer. Blood clearance rate, *in vivo* stability and other advantageous properties are employed in different aspects and embodiments of the invention, e.g. in tumor targeting. The different *in vivo* behavior of different antibody molecule formats may be exploited for different diagnostic and/or therapeutic purposes, depending on clinical needs and disease.

Despite their enormous potential as therapeutic agents, monoclonal antibodies (mAbs) of non-human origin have performed poorly in clinical trials as a result of their immunogenicity (1 Shawlert et al., 1985; 2 Miller et al., 1983), poor pharmacokinetic properties (3 Hakimi et al., 1991; 4 Stephens et al., 1995) and inefficiency in recruiting effector functions (5 Riechmann et al., 1988; 6 Junghens et al., 1990). The recent prospect of isolating human antibody fragments from phage display libraries (7 McCafferty et al., 1990; 8 Lowman et al., 1991; for reviews see 9 Nilsonn et al., 2000 and 10 Winter et al., 1994) transcends these problems, revitalizing studies and rekindling hopes of using these reagents to treat major diseases. Indeed, these molecules should serve as ideal building blocks for novel diagnostic and therapeutic tools (11 Reichert, 2001; 12 Huls et al., 1999). Furthermore, these antibodies can be "matured" to reach affinities in the picomolar range (13 Pini et al., 1998), at least desirable, if not necessary, for their clinical use.

Clinical applications of human antibody fragments for the selective delivery of diagnostic or therapeutic agents nonetheless require highly specific targets. In the case of tumors, the most popular targets are cell-surface antigens, which are usually neither abundant nor stable. Nevertheless, during tumor progression, the microenvironment surrounding tumor cells undergoes extensive modification that generates a "tumoral environment" which represents a target for antibody-based tumor therapy (14 Neri and Zardi, 1998). In fact, the concept that the altered tumor microenvironment is itself a carcinogen that can be targeted is increasingly gaining consensus. Molecules that are able to effectively deliver therapeutic agents to the tumor microenvironment thus represent promising and important new tools for cancer therapy (15 Bissel, 2001; 14 Neri and Zardi, 1998).

Fibronectin is an extracellular matrix (ECM) component that is widely expressed in a variety of normal tissues and body fluids. Different FN isoforms can be generated by the alternative splicing of the FN pre-mRNA, a process that is modulated by cytokines and extracellular pH (16 Balza et al. , 1988; 17 Carnemolla et al., 1989; 18 Borsi et al., 1990; 19 Borsi et al., 1995). The complete type III repeat ED-B, also known as the extratype III repeat B (EIIIB), may be entirely included or omitted in the FN molecule (20 Zardi et al., 1987). ED-B is highly conserved in different species, having 100% homology in all mammalians thus far studied (human, rat, mouse, dog) and 96% homology with a similar domain in chicken. The FN isoform containing ED-B (B-FN) is undetectable immunohistochemically in normal adult tissues, with the exception of tissues undergoing physiological remodelling (e.g., endometrium and ovary) and during wound healing (17 Carnemolla et al., 1989; 21 ffrench-Constant, et al., 1989). By contrast, its expression in tumors and fetal tissues is high (17 Carnemolla et al, 1989). Furthermore, it has been demonstrated that B-FN is a marker of angiogenesis (22 Castellani et al., 1994) and that endothelial cells invading tumor tissues migrate along ECM fibers containing B-FN (23 Tarli et al. 1999).

Selective targeting of tumoral vasculature has been described using a human recombinant antibody, scFv(L19) (13 Pini et al., 98), specific for the B-FN isoform (24 Carnemolla et al., 1996; 23 Tarli et al., 99; 25 Viti et al., 99; 26 Neri et al., 97; 27 Demartis et al., 2001). The antibody may be used in both *in vivo* diagnostic (immunoscintigraphy) and therapeutic approaches entailing the selective delivery of therapeutic radionuclides or toxic agents to tumoral vasculature. In addition, Birchler et al. (28 1999) showed that scFv(L19), chemically coupled to a photosensitizer, selectively accumulates in the newly formed blood vessels of the angiogenic rabbit cornea model and, after irradiation with near infrared light, mediates complete and selective occlusion of ocular neovasculature.

More recently, Nilsson et al. (29 2001) reported that the immunoconjugate of scFv(L19) with the extracellular domain of tissue factor mediates selective infarction in different types of murine tumor models. Furthermore, fusion proteins of scFv(L19) and IL-2 or IL-12 have shown the enhanced therapeutic efficacy of these two cytokines (30 Halin et al.,submitted; 31 Carnemolla et al., 2002). See also WO01/62298 for use of fusions in treatment of lesions of pathological angiogenesis, including tumors. Finally, since L19 reacts equally well with mouse and human ED-B, it can be used for both pre-clinical and clinical studies.

See also PCT/GB97/01412, PCT/EP99/03210, PCT/EP01/02062 and PCT/IB01/00382.

Different antibody formats have shown diverse behavior in terms of *in vivo* stability, clearance and performance in tumor targeting (32 Wu et al., 2000). A mini-immunoglobulin or small immunoprotein (SIP) is described in (33 Li et al., 1997).

The present invention is based on preparation of, characterization of and investigation of the *in vivo* biodistribution of L19 human antibody molecules in different formats, namely, scFv, mini-immunoglobulin and complete IgG1, and labelling with radioisotopes.

*Brief Description of the Figures*

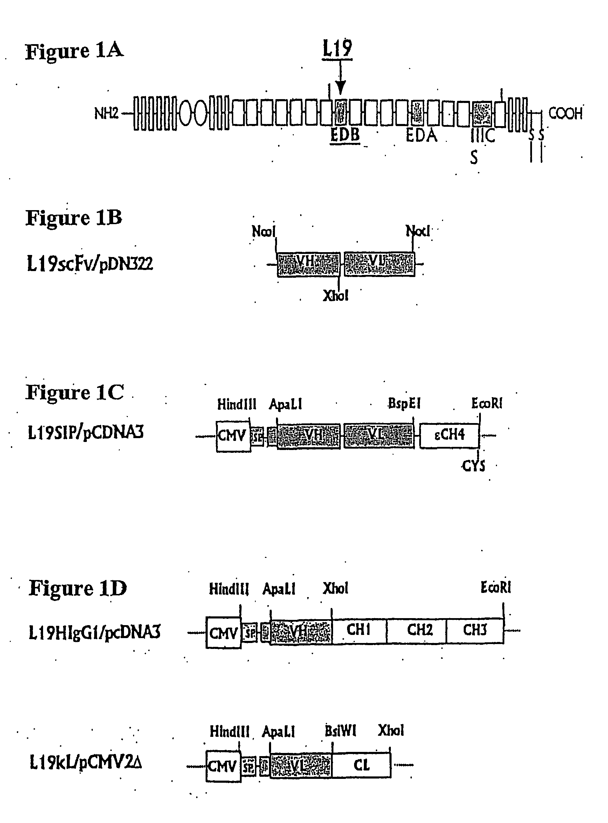

Figure 1 shows models illustrating the structures of different proteins. A: Model of the domain structure of a FN subunit. The protein sequences undergoing alternative splicing are indicated in grey. As indicated, the epitope of the recombinant antibody L19 is localized within the repeat ED-B. B - D: Schemes of the constructs used to express, respectively, L19 (scFv) (B); L19-SIP (C); and L19-IgGI/K.

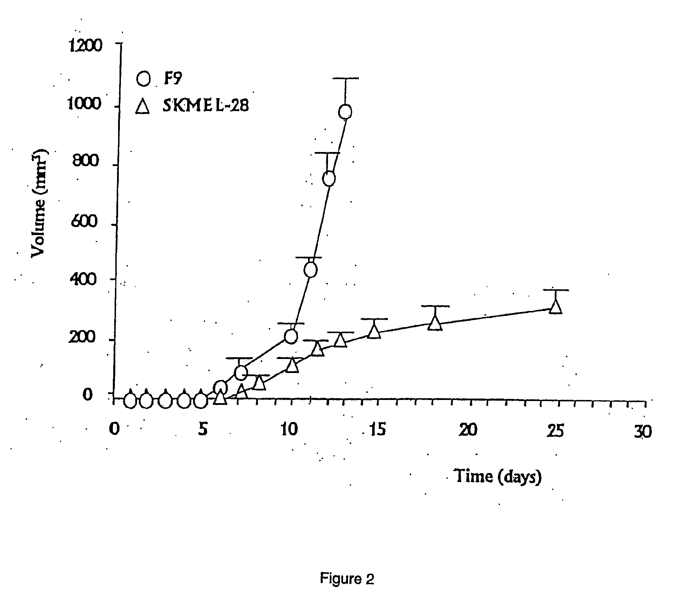

Figure 2 shows growth curves of SK-MEL-28 tumor in nude mice (triangles) and of F9 tumor in 129 mouse strain (circles). The volume (mm$^3$) is plotted versus time (days). Each data point is the average of six mice $\pm$ SD.

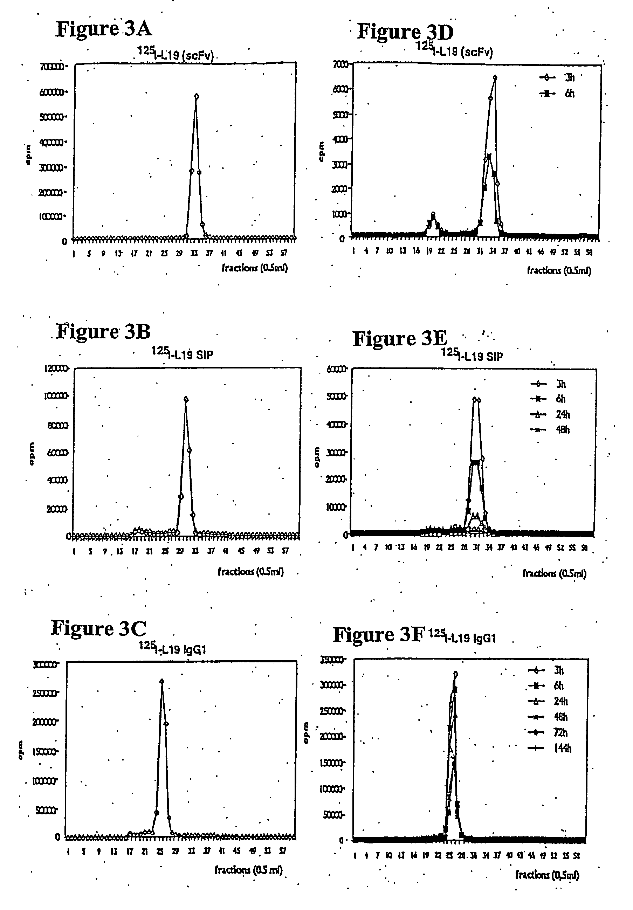

Figure 3 shows the results of size exclusion chromatography on the different L19 formats. In panels A, B and C are shown size exclusion chromatography (Superdex 200) profiles of the L19 formats scFv, mini-immunoglobulin and IgG1, respectively, after radioiodination. Panels D, E and F show size exclusion chromatography (Superdex 200) profiles of plasma at the indicated times after i.v. injection of the radioiodinated L19 formats, scFv, mini-immunoglobulin and IgG1, respectively. No changes in the curve profiles of L19-SIP or L19-IgG1 were detected when loading plasma at different times after injection, while 3h after L19(scFv)2 injection a second peak of higher molecular mass was observed.

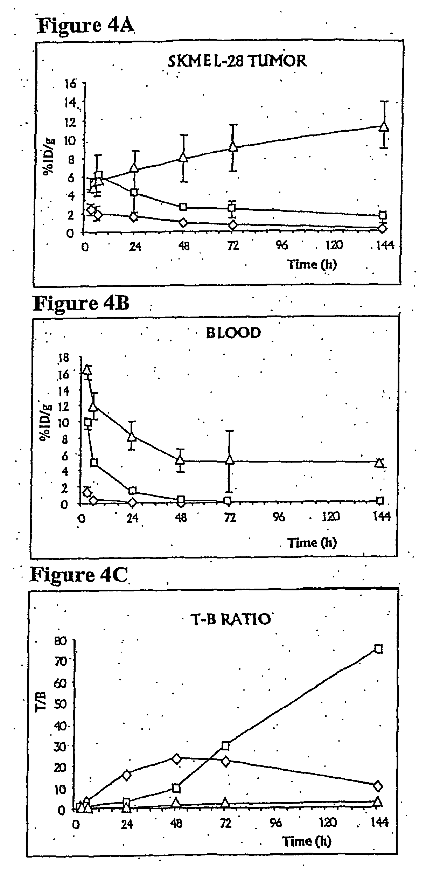

Figure 4 shows results of biodistribution experiments in SK-MEL-28 tumor-bearing mice using different radioiodinated L19 antibody molecule formats. The variations of the %ID/g in the tumor (Figure 4A) and in the blood (Figure 4B) at the indicated times after i.v. injection are reported. In Figure 4C the tumor-blood ratios of the %ID/g are plotted. The curves of L19(scFv) are indicated by diamonds, of L19 mini-immunoglobulin by squares and of L19 IgG1 by triangles.

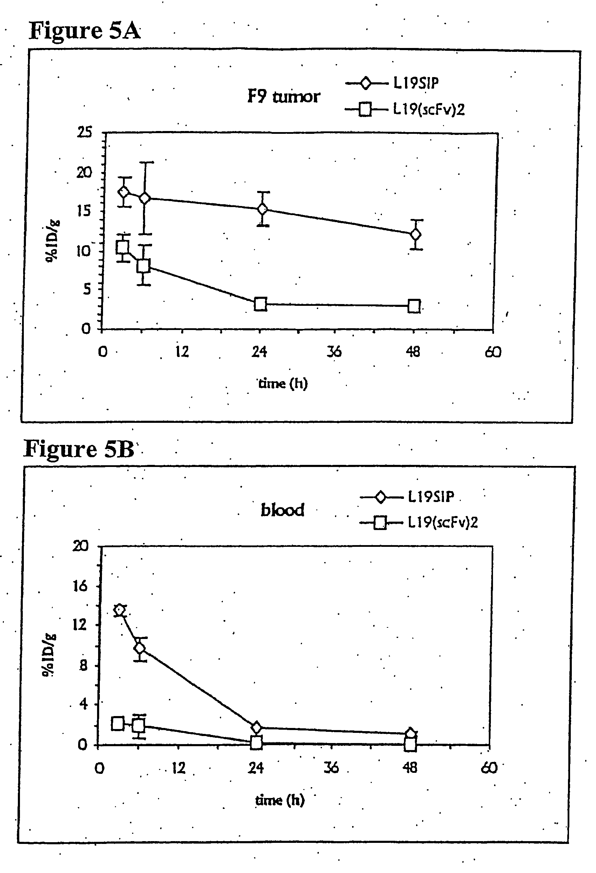

Figure 5 shows results of biodistribution experiments in F9 tumor-bearing mice using radioiodinated L19(scFv) (squares) and L19 mini-immunoglobulin (diamonds). The variations of the %ID/g in the tumor (A) and in the blood (B), at the indicated different times after i.v. injection are reported.

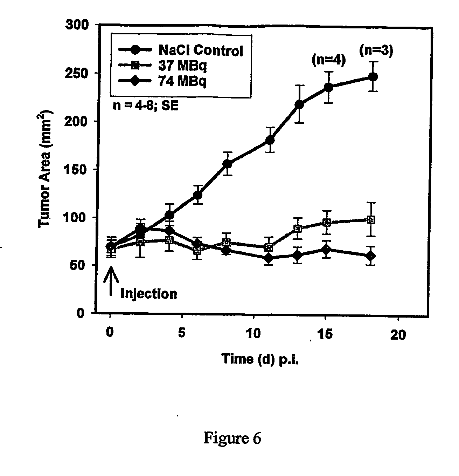

Figure 6 shows change in U251 tumor area (square millimetres) over time (days) post injection of physiological saline and I-131-L19-SIP respectively.

The present invention relates to specific binding members that bind human ED-B of fibronectin, wherein the specific binding members are radiolabelled with one or more isotopes selected from the group consisting of $^{76}$Br, $^{77}$Br, $^{123}$I, $^{124}$I, $^{131}$I and $^{211}$At. The invention also provides methods of producing such specific binding members, and their use in diagnostic and therapeutic applications.

Specific binding members of the invention showed favorable properties in animal experiments, such as higher doses delivered to tumor compared to red marrow, and high tumor accumulation.

In one aspect, the present invention provides a specific binding member which binds human ED-B of fibronectin and which comprises the L19 VH domain and a VL domain, optionally the L19 VL domain, wherein the specific binding member comprises a mini-immunoglobulin comprising said antibody VH domain and antibody VL domain fused to $\varepsilon_{S2}$-CH4 and dimerized or comprises a whole IgG1 antibody molecule, and wherein the specific binding member is radiolabelled with an isotope selected from the group consisting of $^{76}$Br, $^{77}$Br, $^{123}$I, $^{124}$I, $^{131}$I and $^{211}$At. Preferably, the radioisotope is $^{123}$I or $^{131}$I, and most preferably $^{131}$I.

A radiolabel or radiolabelled molecule may be attached to the specific binding member may be labelled at e.g. a tyrosine, lysine or cysteine residue.

The L19 VH domain and L19 VL domain sequences are set out in Pini et al. (1998) J. Biol. Chem. 273: 21769-21776, those sequences being fully incorporated herein by reference to Pini et al. as if set out here.

Generally, a VH domain is paired with a VL domain to provide an antibody antigen binding site. In a preferred embodiment, the L19 VH domain is paired with the L19 VL domain, so that an antibody antigen binding site is formed comprising both the L19 VH and VL domains. In other embodiments, the L19 VH is paired with a VL domain other than the L19 VL. Light-chain promiscuity is well established in the art.

One or more CDRs may be taken from the L19 VH or VL domain and incorporated into a suitable framework.

This is discussed further below. L19 VH CDR's 1, 2 and 3 are shown in SEQ ID NO.'s 1, 2, and 3, respectively. L19 VL CDR's 1, 2 and 3 are shown in SEQ ID NO.'s 1, 2 and 3, respectively.

In a preferred embodiment, the specific binding member is L19-SIP, most preferably $^{123}$I-labelled L19-SIP (herein referred to as I-123-L19-SIP) or $^{131}$I-labelled L19-SIP (herein referred to as I-131-L19-SIP).

Variants of the VH and VL domains and CDRs of which the sequences are set out herein and which can be employed in specific binding members for ED-B can be obtained by means of methods of sequence alteration or mutation and screening.

Variable domain amino acid sequence variants of any of the VH and VL domains whose sequences are specifically disclosed herein may be employed in accordance with the present invention, as discussed. Particular variants may include one or more amino acid sequence alterations (addition, deletion, substitution and/or insertion of an amino acid residue), maybe less than about 20 alterations, less than about 15 alterations, less than about 10 alterations or less than about 5 alterations, 4, 3, 2 or 1. Alterations may be made in one or more framework regions and/or one or more CDR's.

A specific binding member according to the invention may be one which competes for binding to antigen with a specific binding member which both binds ED-B and comprises an antigen-binding site formed of the L19 VH domain and L19 VL domain. Competition between binding members may be assayed easily *in vitro,* for example using ELISA and/or by tagging a specific reporter molecule to one binding member which can be detected in the presence of other untagged binding member(s), to enable identification of specific binding members which bind the same epitope or an overlapping epitope.

Thus, further aspects of the present invention employ a specific binding member comprising a human antibody antigen-binding site which competes with L19 for binding to ED-B.

A specific binding member according to the present invention may bind ED-B with at least the affinity of L19, binding affinity of different specific binding members being compared under appropriate conditions.

In addition to antibody sequences, a specific binding member according to the present invention may comprise other amino acids, e.g. forming a peptide or polypeptide, such as a folded domain, or to impart to the molecule another functional characteristic in addition to ability to bind antigen. Specific binding members of the invention may carry a detectable label, or may be conjugated to a toxin or enzyme (e.g. via a peptidyl bond or linker).

In treatment of disorders or lesions of pathological angiogenesis, a specific binding member of the invention may be conjugated to a toxic molecule, for instance a biocidal or cytotoxic molecule that may be selected from interleukin-2 (IL-2), doxorubicin, interleukin-12 (IL-12), Interferon-$\gamma$ (IFN-$\gamma$), Tumor Necrosis Factor $\alpha$ (TNF$\alpha$) and tissue factor (preferably truncated tissue factor, e.g. to residues 1-219). See e.g. WO01/62298.

Specific binding members according to the invention may be used in a method of treatment or diagnosis of the human or animal body, such as a method of treatment (which may include prophylactic treatment) of a disease or disorder in a human patient which comprises administering to said patient an effective amount of a specific binding member of the invention. Preferably, a specific binding member according to the invention is administered to the patient by parenteral administration. Conditions treatable in accordance with the present invention include tumors, especially solid tumors, and other lesions of pathological angiogenesis, including, rheumatoid arthritis, diabetic retinopathy, age-related macular degeneration, and angiomas.

Specific binding members are well suited for radiolabelling with isotopes selected from the group consisting of $^{76}$Br, $^{77}$Br, $^{123}$I, $^{124}$I, $^{131}$I and $^{211}$At and subsequent use in radiodiagnosis and radiotherapy.

A yet further aspect provides a method of producing a specific binding member of the invention, comprising labelling a specific binding member with a radioisotope selected from the group consisting of $^{76}$Br, $^{77}$Br, $^{123}$I, $^{124}$I, $^{131}$I and $^{211}$At.

To radiolabel the specific binding member directly, tyrosine moieties in the molecule may be targeted. In this particular procedure, the halogenide, e.g. Br⁻, I⁻, At⁻ is oxidised by an appropriate oxidant, e.g. iodogen® (coated tubes), iodo-Beads, chloramine-T (sodium salt of N-chloro-p-toluenesulfonamide) etc. in the presence of the active pharmaceutical ingredient (API).

Indirect labelling with e.g. bromine, iodine or astatine may be performed by pre-labelling a bi-functional halogen carrier, preferably derived from e.g. benzoic acid derivatives, Bolton-Hunter derivatives, benzene derivatives etc. The carrier may be transformed into an activated species to be conjugated to the $\varepsilon$-amino group of Lysine residues or the N-terminus of the API. This indirect method also provides a synthetic route to radiolabel the peptide compounds chemoselectively at the sulfhydryl group of a cysteine moiety. The cysteine bridged molecules may first be reduced by an appropriate reducing agent e.g. stannous(II)chloride, Tris(2-carboxyethyl)phosphine (TCEP) generating free cysteine SH-groups that can react with the halogen carrier. As functional groups for the binding maleimide and $\alpha$-brom acetamide derivatives may be employed.

A method of producing a specific binding member according to the invention may comprise expressing nucleic acid encoding the specific binding member prior to labelling the specific binding member. Thus, as an earlier step, the method of producing the specific binding member may optionally comprise causing or allowing expression from encoding

**EP 2 246 070 A2**

nucleic acid, i.e. nucleic acid comprising a sequence encoding the specific binding member. Such a method may comprise culturing host cells under conditions for production of said specific binding member.

A method of production may comprise a step of isolation and/or purification of the product. The specific binding member may be isolated and/or purified following expression from nucleic acid, and/or recovery from host cells. The isolation and/or purification may be prior to labelling. Alternatively or additionally, the specific binding member may be isolated and/or purified after labelling.

A method of production may comprise formulating the product into a composition including at least one additional component, such as a pharmaceutically acceptable excipient. Thus, the (labelled) specific binding member may be formulated into a composition including at least one additional component such as a pharmaceutically acceptable excipient.

These and other aspects of the invention are described in further detail below.

*TERMINOLOGY*

*Specific binding member*

This describes a member of a pair of molecules which have binding specificity for one another. The members of a specific binding pair may be naturally derived or wholly or partially synthetically produced. One member of the pair of molecules has an area on its surface, or a cavity, which specifically binds to and is therefore complementary to a particular spatial and polar organisation of the other member of the pair of molecules. Thus the members of the pair have the property of binding specifically to each other. Examples of types of specific binding pairs are antigen-antibody, biotin-avidin, hormone-hormone receptor, receptor-ligand, enzyme-substrate. This application is concerned with antigen-antibody type reactions.

*Antibody molecule*

This describes an immunoglobulin whether natural or partly or wholly synthetically produced. The term also covers any polypeptide or protein comprising an antibody binding domain. Antibody fragments which comprise an antigen binding domain are such as Fab, scFv, Fv, dAb, Fd; and diabodies. The present invention is concerned with whole IgG1 antibody molecules and mini-immunoglobulins comprising $\varepsilon_{S2}$-CH4 as disclosed.

Techniques of recombinant DNA technology may be used to produce from an initial antibody molecule other antibody molecules which retain the specificity of the original antibody molecule. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB 2188638A or EP-A-239400.

As antibodies can be modified in a number of ways, the term "antibody molecule" should be construed as covering any specific binding member or substance having an antibody antigen-binding domain with the required specificity. Thus, this term covers antibody fragments and derivatives, including any polypeptide comprising an immunoglobulin antigen-binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023.

*Antigen binding domain*

This describes the part of an antibody molecule which comprises the area which specifically binds to and is complementary to part or all of an antigen. Where an antigen is large, an antibody may only bind to a particular part of the antigen, which part is termed an epitope. An antigen binding domain may be provided by one or more antibody variable domains (e.g. a so-called Fd antibody fragment consisting of a VH domain). Preferably, an antigen binding domain comprises an antibody light chain variable region (VL) and an antibody heavy chain variable region (VH).

*Specific*

This may be used to refer to the situation in which one member of a specific binding pair will not show any significant binding to molecules other than its specific binding partner(s). The term is also applicable where e.g. an antigen binding domain is specific for a particular epitope which is carried by a number of antigens, in which case the specific binding member carrying the antigen binding domain will be able to bind to the various antigens carrying the epitope.

5

*Comprise*

This is generally used in the sense of include, that is to say permitting the presence of one or more features or components.

*Isolated*

This refers to the state in which specific binding members of the invention, or nucleic acid encoding such binding members, will generally be in accordance with the present invention. Members and nucleic acid will be free or substantially free of material with which they are naturally associated such as other polypeptides or nucleic acids with which they are found in their natural environment, or the environment in which they are prepared (e.g. cell culture) when such preparation is by recombinant DNA technology practised *in vitro* or *in vivo.* Members and nucleic acid may be formulated with diluents or adjuvants and still for practical purposes be isolated - for example the members will normally be mixed with gelatin or other carriers if used to coat microtitre plates for use in immunoassays, or will be mixed with pharmaceutically acceptable carriers or diluents when used in diagnosis or therapy. Specific binding members may be glycosylated, either naturally or by systems of heterologous eukaryotic cells (e.g. CHO or NS0 (ECACC 85110503) cells, or they may be (for example if produced by expression in a prokaryotic cell) unglycosylated.

By "substantially as set out" it is meant that the relevant CDR or VH or VL domain of the invention will be either identical or highly similar to the specified regions of which the sequence is set out herein. By "highly similar" it is contemplated that from 1 to 5, preferably from 1 to 4 such as 1 to 3 or 1 or 2, or 3 or 4, substitutions may be made in the CDR and/or VH or VL domain.

The structure for carrying a CDR of the invention will generally be of an antibody heavy or light chain sequence or substantial portion thereof in which the CDR is located at a location corresponding to the CDR of naturally occurring VH and VL antibody variable domains encoded by rearranged immunoglobulin genes. The structures and locations of immunoglobulin variable domains may be determined by reference to (Kabat, E.A. et al, Sequences of Proteins of Immunological Interest. 5th Edition. US Department of Health and Human Services. 1991, and updates thereof, now available on the Internet (http://immuno.bme.nwu.edu or find "Kabat" using any search engine).

Preferably, a CDR amino acid sequence substantially as set out herein is carried as a CDR in a human variable domain or a substantial portion thereof. The L19 VH CDR3 and/or L19 VL CDR3 sequences substantially as set out herein may be used in preferred embodiments of the present invention and it is preferred that each of these is carried as a CDR3 in a human heavy or light chain variable domain, as the case may be, or a substantial portion thereof.

A substantial portion of an immunoglobulin variable domain will comprise at least the three CDR regions, together with their intervening framework regions. Preferably, the portion will also include at least about 50% of either or both of the first and fourth framework regions, the 50% being the C-terminal 50% of the first framework region and the N-terminal 50% of the fourth framework region. Additional residues at the N-terminal or C-terminal end of the substantial part of the variable domain may be those not normally associated with naturally occurring variable domain regions. For example, construction of specific binding members of the present invention made by recombinant DNA techniques may result in the introduction of N- or C-terminal residues encoded by linkers introduced to facilitate cloning or other manipulation steps. Other manipulation steps include the introduction of linkers to join variable domains of the invention to further protein sequences including immunoglobulin heavy chains, other variable domains or protein labels as discussed in more details below.

In an IgG1 antibody molecule according to the present invention, VL domains may be attached at the C-terminal end to antibody light chain constant domains including human $C\kappa$ or $C\lambda$ chains, preferably $C\kappa$ chains.

In addition to being labelled with $^{76}Br$, $^{77}Br$, $^{123}I$, $^{124}I$, $^{131}I$ and/or $^{211}At$, specific binding members of the invention may be labelled with a second detectable or functional label. Detectable labels are described below and include radiolabels such as radioisotopes of Technetium, Indium, Yttrium, Copper, Lutetium or Rhenium, in particular $^{94m}Tc$, $^{99m}Tc$, $^{186}Re$, $^{188}Re$, $^{111}In$, $^{86}Y$, $^{88}Y$, $^{177}Lu$, $^{64}Cu$ and $^{67}Cu$, which may be attached to antibodies of the invention using conventional chemistry known in the art of antibody imaging as described herein. Other radioisotopes that may be used include $^{203}Pb$, $^{67}Ga$, $^{68}Ga$, $^{43}Sc$, $^{47}Sc$, $^{110m}In$, $^{97}Ru$, $^{62}Cu$, $^{68}Cu$, $^{86}Y$, $^{88}Y$, $^{90}Y$, $^{121}Sn$, $^{161}Tb$, $^{153}Sm$, $^{166}Ho$, $^{105}Rh$, $^{177}Lu$, $^{72}Lu$ and $^{18}F$.

Labels also include enzyme labels such as horseradish peroxidase. Labels further include chemical moieties such as biotin which may be detected via binding to a specific cognate detectable moiety, e.g. labelled avidin.

An example labelling protocol is as follows:

To radiolabel the specific binding members directly, the cysteine bridged molecules are first reduced by an appropriate reducing agent e.g. stannous(II)chloride, Tris(2-carboxyethyl)phosphine (TCEP) generating free cysteine SH-groups that can react with isotopes e.g. Tc or Re. In this particular procedure, the permetalates obtained from an instant generator system are reduced by a reducing agent e.g. stannous(II)chloride in the presence of an auxillary ligand e.g. sodium tartrate and the API (details are provided below in the experimental section).

Indirect labeling with e.g. indium, yttrium, lanthanides or technetium and rhenium may be performed by pre-conjugating a chelating ligand, preferably derived from ethylene diamine tetraacetic acid (EDTA), diethylene triamine pentaacetic acid (DTPA), cyclohexyl 1,2-diamine tetraacetic acid (CDTA), ethyleneglycol-O,O'-bis(2-aminoethyl)-N,N, N',N'-diacetic acid (HBED), triethylene tetraamine hexaacetic acid (TTHA), 1,4,7,10-tetraazacyclododecane-N,N',N"'-tetraacetic acid (DOTA), 1,4,7-triazacyclononane-N,N',N"-triacetic acid (NOTA), 1,4,8,11-tetraazacyclotetradecane-N, N',N",N"'-tetraacetic acid (TETA), mercaptoacetyl diglycine (MAG$_2$), mercaptoacetyl triglycine (MAG$_3$), mercaptoacetyl glycyl cysteine (MAGC), cysteinyl glycyl cysteine (CGC) to either amine or thiol groups of the specific binding member. The chelating ligands possess a suitable coupling group e.g. active esters, maleimides, thiocarbamates or $\alpha$-halogenated acetamide moieties. For conjugating chelating ligands to amine groups e.g. $\epsilon$-NH$_2$-groups of lysine residues previous reduction of the L-19-SIP compound is not required.

Methods of labelling a specific binding member may comprise conjugating an activated bi-functional halogen carrier containing a radioiosotope selected from the group consisting of $^{76}$Br, $^{77}$Br, $^{123}$I, $^{124}$I, $^{131}$I and $^{211}$At to a lysine residue or N terminus, and to a cysteine residue of the specific binding member. The method may comprise conjugating the halogen carrier to a lysine or cysteine residue of the specific binding member, or to the N terminus of the specific binding member. Either or both of (i) a cysteine residue and (ii) a lysine residue or the N terminus, may be labelled with the same or a different radioisotope according to the invention.

Specific binding members of the present invention are designed to be used in methods of diagnosis or treatment in human or animal subjects, preferably human. The specific binding members are especially suitable for use in methods of radiotherapy and radiodiagnosis.

Accordingly, further aspects of the invention provide methods of treatment comprising administration of a specific binding member as provided, pharmaceutical compositions comprising such a specific binding member, and use of such a specific binding member in the manufacture of a medicament for administration, for example in a method of making a medicament or pharmaceutical composition comprising formulating the specific binding member with a pharmaceutically acceptable excipient.

Clinical indications in which a specific binding member of the invention may be used to provide therapeutic benefit include tumors such as any solid tumor, also other lesions of pathological angiogenesis, including rheumatoid arthritis, diabetic retinopathy, age-related macular degeneration, and angiomas.

Specific binding members according to the invention may be used in a method of treatment of the human or animal body, such as a method of treatment (which may include prophylactic treatment) of a disease or disorder in a human patient which comprises administering to said patient an effective amount of a specific binding member of the invention. Preferably, the treatment is radiotherapy. Conditions treatable in accordance with the present invention are discussed elsewhere herein.

Specific binding members according to the invention may be used in SPECT imaging, PET imaging and therapy. Preferred isotopes for SPECT imaging include $^{123}$I and $^{131}$I. A preferred isotope for PET is $^{124}$I. $^{131}$I is a preferred isotope for use in therapy.

Due to the use of different isotopes of one element for imaging and therapy the biodistribution of the respective immunoconjugates is identical. This is an advantage compared with other approaches which are using $^{111}$In-labeled derivatives for imaging in order to predict the biodistribution of the respective $^{90}$Y-labeled therapeutic derivatives because the biodistribution of corresponding $^{111}$In and $^{90}$Y labeled derivatives could be different; see Carrasquillo J.A. et al. (1999) J Nucl Med 40: 268-276.

Accordingly, further aspects of the invention provide methods of treatment comprising administration of a specific binding member as provided, pharmaceutical compositions comprising such a specific binding member, and use of such a specific binding member in the manufacture of a medicament for administration, for example in a method of making a medicament or pharmaceutical composition comprising formulating the specific binding member with a pharmaceutically acceptable excipient.

In accordance with the present invention, compositions provided may be administered to individuals. Administration is preferably in a "therapeutically effective amount", this being sufficient to show benefit to a patient. Such benefit may be at least amelioration of at least one symptom. The actual amount administered, and rate and time-course of administration, will depend on the nature and severity of what is being treated. Prescription of treatment, e.g. decisions on dosage etc, is within the responsibility of general practitioners and other medical doctors. Appropriate doses of antibody are well known in the art; see Ledermann J.A. et al. (1991) Int J. Cancer 47: 659-664; Bagshawe K.D. et al. (1991) Antibody, Immunoconjugates and Radiopharmaceuticals 4: 915-922.

A composition may be administered alone or in combination with other treatments, either simultaneously or sequentially dependent upon the condition to be treated.

Specific binding members of the present invention, including those comprising an antibody antigen-binding domain, may be administered to a patient in need of treatment via any suitable route, usually by injection into the bloodstream and/or directly into the site to be treated, e.g. tumor. Preferably, the specific binding member is parenterally administered. The precise dose will depend upon a number of factors, the route of treatment, the size and location of the area to be treated (e.g. tumor), the precise nature of the antibody (e.g. whole IgG1 antibody molecule, mini-immunoglobulin molecule), and the nature of any detectable label or other molecule attached to the antibody molecule. A typical antibody dose will be in the range 10-50 mg.

This is a dose for a single treatment of an adult patient, which may be proportionally adjusted for children and infants, and also adjusted for other antibody formats in proportion to molecular weight. Treatments may be repeated at daily, twice-weekly, weekly or monthly intervals, at the discretion of the physician.

Specific binding members of the present invention will usually be administered in the form of a pharmaceutical composition, which may comprise at least one component in addition to the specific binding member.

Thus pharmaceutical compositions according to the present invention, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous.

For intravenous, injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A composition may be administered alone or in combination with other treatments, either simultaneously or sequentially dependent upon the condition to be treated. Other treatments may include the administration of suitable doses of pain relief drugs such as non-steroidal anti-inflammatory drugs (e.g. aspirin, paracetamol, ibuprofen or ketoprofen) or opiates such as morphine, or anti-emetics.

The present invention provides a method comprising causing or allowing binding of a specific binding member as provided herein to ED-B. As noted, such binding may take place *in vivo*, e.g. following administration of a specific binding member, or nucleic acid encoding a specific binding member, or it may take place *in vitro,* for example in ELISA, Western blotting, immunocytochemistry, immuno-precipitation or affinity chromatography.

The amount of binding of specific binding member to ED-B may be determined. Quantitation may be related to the amount of the antigen in a test sample, which may be of diagnostic interest, which may be of diagnostic interest.

The reactivities of antibodies on a sample may be determined by any appropriate means. Radioimmunoassay (RIA) is one possibility. Radioactive labelled antigen is mixed with unlabelled antigen (the test sample) and allowed to bind to the antibody. Bound antigen is physically separated from unbound antigen and the amount of radioactive antigen bound to the antibody determined. The more antigen there is in the test sample the less radioactive antigen will bind to the antibody. A competitive binding assay may also be used with non-radioactive antigen, using antigen or an analogue linked to a reporter molecule. The reporter molecule may be a fluorochrome, phosphor or laser dye with spectrally isolated absorption or emission characteristics. Suitable fluorochromes include fluorescein, rhodamine, phycoerythrin and Texas Red. Suitable chromogenic dyes include diaminobenzidine.

Other reporters include macromolecular colloidal particles or particulate material such as latex beads that are coloured, magnetic or paramagnetic, and biologically or chemically active agents that can directly or indirectly cause detectable signals to be visually observed, electronically detected or otherwise recorded. These molecules may be enzymes which catalyse reactions that develop or change colours or cause changes in electrical properties, for example. They may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed.

The signals generated by individual antibody-reporter conjugates may be used to derive quantifiable absolute or relative data of the relevant antibody binding in samples (normal and test).

The present invention further extends to a specific binding member which competes for binding to ED-B with any specific binding member which both binds the antigen and comprises a V domain including a CDR with amino acid substantially as set out herein, preferably a VH domain comprising VH CDR3 of SEQ ID NO. 3. Competition between binding members may be assayed easily *in vitro,* for example by tagging a specific reporter molecule to one binding member which can be detected in the presence of other untagged binding member(s), to enable identification of specific binding members which bind the same epitope or an overlapping epitope. Competition may be determined for example using the ELISA as described in Carnemolla et al. (24 1996).

As stated above, methods of producing specific binding members according to the invention may comprise expressing encoding nucleic acid, and may optionally involve culturing host cells under conditions for production of the specific binding member. Specific binding members and encoding nucleic acid molecules and vectors according to or for use in the present invention may be provided isolated and/or purified, e.g. from their natural environment, in substantially pure or homogeneous form, or, in the case of nucleic acid, free or substantially free of nucleic acid or genes origin other than the sequence encoding a polypeptide with the required function.

Nucleic acid used according to the present invention may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells, NSO mouse melanoma cells and many others. A common, preferred bacterial host is *E. coli.*

The expression of antibodies and antibody fragments in prokaryotic cells such as *E. coli* is well established in the art. For a review, see for example Plückthun, A. Bio/Technology 9: 545-551 (1991). Expression in eukaryotic cells in culture is also available to those skilled in the art as an option for production of a specific binding member, see for recent reviews, for example Ref, M.E. (1993) Curr. Opinion Biotech. 4: 573-576; Trill J.J. et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, Molecular Cloning: a Laboratory Manual: 3nd edition, Sambrook et al., 2001, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992. The disclosures of Sambrook et al. and Ausubel et al. are incorporated herein by reference.

A method of producing a specific binding member according to the invention may further comprise introducing encoding nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage.

The introduction may be followed by causing or allowing expression from the nucleic acid, e.g. by culturing host cells under conditions for expression of the gene.

In one embodiment, the nucleic acid of the invention is integrated into the genome (e.g. chromosome) of the host cell. Integration may be promoted by inclusion of sequences which promote recombination with the genome, in accordance with standard techniques.

Further aspects and embodiments of the present invention will be apparent to those skilled in the art in the light of the present disclosure including the following experimental exemplification. Methods for synthesis and labelling of the specific binding members of the present invention are more fully illustrated in the following examples. These examples are shown by way of illustration and not by way of limitation.All documents mentioned anywhere in this specification and incorporated by reference.

## *EXPERIMENTAL EXEMPLIFICATION OF ASPECTS AND EMBODIMENTS OF THE PRESENT INVENTION*

### *1. Preparation and characterisation of specific binding members according to the present invention*

The following examples use radiolabelled peptide compounds L19-SIP.

### *1.1 Synthesis of I-131-L19-SIP (Chloramine-T Method)*

200 μg L19-SIP in 230μl PBS (0.2 M PBS, pH 7.4) were placed in a reaction vial, mixed with 185 MBq [$^{131}$I] NaI, and reacted with 30 μL of a freshly prepared solution of Chloramine-T (2 mg/mL) in 0.2 M PBS (pH 7.4). After 1 min, 50 μL of a solution of $Na_2S_2O_5$ (10 mg/mL in PBS 0.2 M, pH 7.4). $^{131}$I-labeled L19-SIP was purified by gel-chromatography using a NAP-5 column (Amersham, Eluent: PBS), pre-blocked with 5 ml of 0.5 % bovine serum albumin in PBS.

| | |
|---|---|
| Radiochemical yield: | 45.7 %. |
| Radiochemical purity: | 88.3 % (SDS-PAGE). |
| Specific activity: | 31.7 MBq/nmol. |

(continued)

| | |
|---|---|
| Immunoreactivity: | 76 % |

### 1.2 Synthesis of I-131-L19-SIP (iodogen method)

800 µg L19-SIP in 800 µl PBS (0.2 M PBS, pH 7.4) and 500 MBq [$^{131}$I]NaI were mixed and placed in a reaction vial (iodogen tube, Pierce Inc.). The mixture was shaken gently, over a period of 30 min at room temperature. $^{131}$I-labeled L19-SIP was purified by gel-chromatography using a NAP-5 column (Amersham, Eluent: PBS), pre-blocked with 5 ml of 0.5 % bovine serum albumin in PBS.

| | |
|---|---|
| Radiochemical yield: | 93.2 %. |
| Radiochemical purity: | 91.1 % (SDS-PAGE). |
| Specific activity: | 46.6 MBq/nmol. |
| Immunoreactivity: | 78 % |

### 1.3 Synthesis of I-123-L19-SIP (iodogen method)

200 µg L19-SIP in 230 µl PBS (0.2 M PBS, pH 7.4) and 200 MBq [$^{123}$I]NaI were mixed and placed in a reaction vial (iodogen tube, Pierce Inc.). The mixture was shaken gently, over a period of 30 min at room temperature. $^{123}$I-labeled L19-SIP was purified by gel-chromatography using a NAP-5 column (Amersham, Eluent: PBS), pre-blocked with 5 ml of 0.5 % bovine serum albumin in PBS.

| | |
|---|---|
| Radiochemical yield: | 81.6 %. |
| Radiochemical purity: | 89.6 % (SDS-PAGE). |
| Specific activity: | 61.2 MBq/nmol. |
| Immunoreactivity: | 84 % |

### 1.4 Synthesis of I-124-L19-SIP (iodogen method)

200 µg L19-SIP in 230 µl PBS (0.2 M PBS, pH 7.4) and 50 MBq [$^{123}$I]NaI were mixed and placed in a reaction vial (iodogen tube, Pierce Inc.). The mixture was shaken gently, over a period of 30 min at room temperature. $^{124}$I-labeled L19-SIP was purified by gel-chromatography using a NAP-5 column (Amersham, Eluent: PBS), pre-blocked with 5 ml of 0.5 % bovine serum albumin in PBS.

| | |
|---|---|
| Radiochemical yield: | 84.5 %. |
| Radiochemical purity: | 89.6 % (SDS-PAGE). |
| Specific activity: | 22.8 MBq/nmol. |
| Immunoreactivity: | 86 % |

### 1.5 Synthesis of (3-(4-hydroxy-3-[$^{131}$I]iodo-phenyl)-propionate)-L19-SIP

500 µg (3-(4-hydroxy-phenyl)-N-(sulfonato-succinimidyl) propionate) was dissolved in 1 mL of DMSO. Ten microliters of Chloramine-T (5 mg/ml in PBS) were mixed with 74 MBq [$^{131}$I]NaI, neutralized with 15 µL of PBS (0.2 M, pH 7.4). One microliter of the (3-(4-hydroxy-phenyl)-N-(sulfonato-succinimidyl) propionate) solution is added to the Chloramine-T/[$^{131}$I]NaI solution and the mixture is allowed to react for 1 min. The reaction is stopped by the addition of 40 µL of a solution of $Na_2S_2O_5$ (10 mg/mL in PBS 0.2 M, pH 7.4), followed by the immediate addition of 200 µg L19-SIP in 230 µl borate buffer (0.2 M PBS, pH 8.5).

(3-(4-hydroxy-3-[$^{131}$I] iodo-phenyl)-propionate)-L19-SIP was purified by gel-chromatography using a NAP-5 column (Amersham, Eluent: PBS), pre-blocked with 5 ml of 0.5 % bovine serum albumin in PBS.

| | |
|---|---|
| Radiochemical yield: | 37.2 %. |
| Radiochemical purity: | 94.6 % (SDS-PAGE). |
| Specific activity: | 10.3 MBq/nmol. |
| Immunoreactivity: | 69 % |

*1.6 MIRD calculations of I-131-L19-SIP*

Based on biodistribution data in tumor-bearing mice absorbed doses of the I-131 labeled L19-SIP could be calculated by the MIRD formalism. Biokinetic modeling was performed with the % ID data of I-131-L19-SIP in human glioblastoma (U251) bearing mice. The residence times were calculated as the areas under the curve of bi- and mono-exponential functions integrated from zero to infinity including the biological and physical half-life of the compound.

Considering the mouse organs as both the radiation source and the radiation target absorbed organ doses as self-to-self doses (no radiation cross fire) could be estimated for I-131-L19-SIP using S-values from the MIRDOSE 3.1 software.

Mouse organ doses (mGy/ MBq):

| | |
|---|---|
| Liver | 50 |
| Kidneys | 160 |
| Spleen | 50 |
| Lung | 220 |
| Ovaries | 180-410 (depending on ovulation cycle status and ED-B expression) |
| Uterus | 600 (depending on ovulation cycle status and ED-B expression) |
| Testis | 55 |
| Blood | 130 |
| Red Marrow | 50 (calculation based on the blood dose) |
| Tumor | 940 (calculated for a 100 mg tumor) |

Using the calculated residence times in the MIRDOSE 3.1 program human absorbed doses can be estimated for the I-131-L19-SIP.

Human organ doses (mGy/ MBq):

| | |
|---|---|
| Adrenals | 9.46E-02 |
| Brain | 1.64E-02 |
| Breasts | 7.33E-02 |
| Gall bladder | 1.00E-01 |
| LLI Wall | 4.47E-01 |
| Small Intestine | 1.10E-01 |
| Stomach | 9.45E-02 |
| ULI Wall | 2.11E-01 |
| Heart Wall | 6.22E-02 |
| Kidneys | 1.86E-01 |
| Liver | 7.46E-02 |
| Lungs | 7.04E-02 |
| Muscle | 8.71E-02 |
| Ovaries | 8.07E-01 |
| Pancreas | 1.15E-01 |
| Red Marrow | 9.11E-02 |
| Bone Surface | 9.74E-02 |
| Skin | 7.20E-02 |
| Spleen | 7.11E-02 |
| Testes | 2.38E-01 |
| Thymus | 8.55E-02 |
| Thyroid | 8.54E-02 |

(continued)

| | |
|---|---|
| Urin Bladder Wall | 7.19E-01 |
| Uterus | 4.72E-01 |
| Total Body | 8.78E-02 |
| EFF DOSE EQUIV | 3.60E-01 |
| EFF DOSE | 3.21E-01 |

It was concluded that the Red Marrow and the reproductive organs (ovaries/ uterus and testes) would be the dose limiting organs. Nevertheless, the therapeutic window based on the dosimetric calculations looked favorable and promising. A Tumor dose to Red Marrow dose ratio of 18 was found. Thus, I-131-L19-SIP displayed a remarkable 18-fold higher dose delivered to the tumor than to the red marrow.

*1.7 Tumor treatment study after single i.v. injection of I-131-L19-SIP into tumor bearing nude mice*

I-131-L19-SIP was injected once intraveneously into U251 (glioblastoma) bearing nude mice (body weight about 27 g). The investigated doses were 37 MBq and 74 MBq, respectively. In addition, a control group of animals (injected once with physiological saline) was investigated. During the days after injection, the tumor size (given in mm$^2$) was determined using a caliper.

The growth of U251 tumors in nude monitored after single intravenous injection of physiological saline and I-131-L19-SIP, respectively, is shown in Figure 6.

A single injection of the I-131-L19-SIP with 74 MBq per animal showed a pronounced effect on the growth of U251-tumors resulting in a stasis for 18 days. The same was true for the low dose group (37 MBq) except a slight tumor growth which was started during the last 5 days for the low dose group. In contrast, tumors of the control group grew continuously during the whole observation period.

The results of this investigation show excellent potential of I-131-L19-SIP for the treatment of solid tumors.

*1.8 Imaging of I-123-L19-SIP after single i.v. injection into tumor bearing nude mice*

The substance of the invention was injected intraveneously in a dose of about 9.25 MBq into F9 (teratocarcinoma) bearing nude mice (body weight about 25 g). Gamma-camera imaging was carried out at various times after administration of the substance.

By planar scintigraphy of I-123-L19-SIP in F9 (teratocarcinoma) bearing nude mice 4 hours after injection and 24 hours after injection, the tumor could be clearly depicted. At 4 hours after injection, besides the strong uptake in the tumor only a slight background in the rest of the body (not bound to a particular organ but derived from the blood pool) could be detected. Whereas the signal in the tumor maintained, the background signal in the rest of the body disappeared over time. Thus, at 24 hours post injection only the tumor could be detected.

The results of this investigation shows the excellent potential of I-123-L19-SIP for the imaging of solid tumors.

*2. Further Examples and Experiments*

*MATERIALS AND METHODS*

*Preparation and expression of scFv, small immunoprotein (SIP) and IgGI constructs scFv*

The scFv(L19) (Figure 1A) is an affinity matured (Kd=5.4x10$^{-11}$M) antibody fragment specifically directed against the ED-B domain of fibronectin (13 Pini et al., 1998). The scFv(D1.3) (7 McCafferty et al.; 26 Neri et al., 1997), a mouse-anti-hen egg white lysozyme scFv, was used as a control. These scFvs were expressed in *E. Coli* strain HB2151 (Maxim Biotech, San Francisco CA) according to Pini et al. (34 1997).

Mini-immunoglobulin

To construct the L19 small immunoprotein (L19-SIP) gene (Figure 1C) the DNA sequence coding for the scFv (L19) was amplified by Polymerase Chain Reaction (PCR) using Pwo DNA Polymerase (Roche), according to manufacturer's recommendations, with primers BC-618 (gtgtgcactcggaggtgcagctgttggagtctggg - SEQ ID NO. 8) and BC-619 (gcctccggatttgatttccaccttggtcccttggcc - SEQ ID NO. 9), containing ApaLI and BspEI restriction sites, respectively. The amplification product was inserted ApaLI/BspEI in the pUT-εSIP vector, which provides the scFv gene with a secretion signal, required for secretion of proteins in the extracellular medium. The pUT-εSIP vector was obtained from the previously described pUT-SIP-long (33 Li et al., 1997) after substituting the human constant γ1-CH3 domain with the CH4 domain of the human IgE secretory isoform IgE-S2 ($\varepsilon_{S2}$-CH4; 35 Batista et al., 1996). CH4 is the domain that allows dimerization in the IgE molecule and the $\varepsilon_{S2}$ isoform contains a cysteine at the carboxyterminal end, which stabilizes the IgE dimer through an inter-chain disulphide bond. In the final SIP molecule the ScFv(L19) was connected to the $\varepsilon_{S2}$-CH4 domain by a short GGSG linker. The SIP gene was then excised from the plasmid pUT-εSIP-L19 with HindIII and EcoRI restriction enzymes and cloned into the mammalian expression vector pcDNA3 (Invitrogen, Groningen, The Netherlands), which contains the Cytomegalovirus (CMV) promoter, in order to obtain the construct pcDNA3-L19-SIP.

The DNA sequence coding for scFv(D1.3) was amplified using the primers BC-721 (ctcgtgcactcgcaggtgcagctgcaggagtca - SEQ ID NO. 10) and BC-732 (ctctccggaccgtttgatctcgcgcttggt - SEQ ID NO. 11) and inserted ApaLI/BspEI in the pUT-εSIP vector. The D1.3-SIP gene was then excised from the pUT-eSIP-D1.3 with HindIII and EcoRI restriction enzymes and cloned into pcDNA3, in order to obtain the construct pcDNA3-D1.3-SIP.

These constructs were used to transfect SP2/0 murine myeloma cells (ATCC, American Type Culture Collection, Rockville, MD, USA) using FuGENE 6 Transfection Reagent (Roche), following the protocol for adherent cells, optimized by the manufacturer. Transfectomas were grown in DMEM supplemented with 10% FCS and selected using 750 μg/ml of Geneticin (G418, Calbiochem, San Diego, CA).

IgG1

To prepare complete IgG1, the variable region of the L19 heavy chain (L19-VH), together with its secretion peptide sequence, was excised with HindIII and XhoI from the previously described L19-pUTεSIP and inserted in the pUC-IgG1 vector, containing the complete human γ1 constant heavy chain gene. The recombinant IgG1 gene was then excised from the pUC-IgG1-L19-VH with HindIII and EcoRI and cloned into pcDNA3, to obtain the construct pcDNA3-L19-IgG1.

For the preparation of the complete L19 light chain, L19-VL was amplified from the L19-pUT-εSIP (described above) by PCR using the primers BC-696 (tggtgtgcactcggaaattgtgttgacgcagtc - SEQ ID NO. 12) and BC-697 (ctctcgtacgtttgatttccaccttggtcc - SEQ ID NO. 13), containing ApaLI and BsiWI restriction sites, respectively. After digestion with ApaLI and BsiWI, the amplification product was inserted in the vector pUT-SEC-hCκ containing the secretion signal sequence and the sequence of the human constant κ light chain. The recombinant light chain gene was then excised from pUT-SEC-hCκ-L19-VL with HindIII and XhoI and inserted in the pCMV2Δ mammalian expression vector, derived from a pcDNA3 vector by removing the resistance gene to G418, to obtain the construct pCMV2Δ-L19-κ.

Equimolar amounts of these constructs were used to cotransfect SP2/0 murine myeloma cells as described above. Geneticin selected clones were screened in ELISA for the ability to secrete chimeric immunoglobulin, complete of heavy and light chains.

All DNA constructs were purified using the Maxiprep system from Qiagen (Hilden, Germany), and the DNA sequences of both strands of the constructs were confirmed using the ABI PRISM dRhodamine Terminator Cycle Sequencing Ready Reaction Kit (Perkin Elmer, Foster City, CA). All restriction enzymes (RE) were from Roche Diagnostics (Milan, Italy), with the exception of BsiWI (New England Biolabs, Beverly, MA). After RE digestion, inserts and vectors were recovered from agarose gels using the Qiaquick method (Qiagen).

*Purification and quality control of antibodies*

Immunoaffinity chromatography was performed to purify the different antibodies according to the procedure described by Carnemolla et al. (24 1996).

ED-B conjugated to Sepharose 4B (Amersham Pharmacia Biotech., Uppsala, Sweden) following manufacturer's instructions (24 Carnemolla et al., 96) was used to immunopurify all different L19 antibody formats, while a column of hen egg white lysozyme (Sigma, St.Louis, USA) conjugated to Sepharose 4B (Amersham Pharmacia) was used for D1.3 antibodies.

The immunopurified antibody formats L19-SIP and L19-IgG1 required no further purification and were dialyzed against PBS, pH 7.4, at +4°C. Since scFvs obtained from immunoaffinity chromatography are made up of two forms, monomeric and dimeric, a second purification step, as described by Demartis et al. (27 2001), was required to isolate the latter form. Batches of the different antibody formats were prepared and analyzed using SDS-PAGE under reducing and non-reducing conditions, immunohistochemistry, size exclusion chromatography (Superdex 200, Amersham Pharmacia Biotech) and ELISA experiments.

*Sodium Dodecyl Sulfate-Polyacrylamide Gel Electrophoresis (SDS-PAGE), Enzyme Linked Immunoabsorbent Assay (ELISA), size exclusion chromatography and immunohistochemistry* Screening ELISA experiments on the conditioned culture media were performed according to Carnemolla et al. (24 1996). To reveal the expression of the different L19 antibody formats, the recombinant fragment 7B89 (24 Carnemolla et al., 1996), containing the ED-B domain of FN, that includes the epitope recognized by the L19, was immobilized on Maxisorp immunoplates (Nunc, Roskilde, Denmark). To detect D1.3 antibodies in ELISA experiments, hen egg white chicken lysozyme (Sigma) was immobilized on NH2 surface EIA plates (Costar, Cambridge, MA). A peroxidase-conjugated rabbit anti human IgE (Pierce, Rockford, IL), diluted according to manufacturer's recommendations, was used as secondary antibody to detect SIPs. A peroxidase-conjugated rabbit anti human IgG (Pierce) was used in the case of IgG1. For the scFvs containing the tag sequence FLAG, a mouse anti-human FLAG monoclonal antibody (M2, Kodak) and a peroxidase-conjugated goat anti-mouse antibody (Pierce) were used as secondary and tertiary antibodies, respectively. In all cases the immunoreactivity with the immobilized antigen was detected using the substrate ABTS for peroxidase (Roche) and photometric absorbance at 405 nm was measured.

A Superdex 200 (Amersham Pharmacia) chromatography column was used to analyze the gel filtration profiles of the purified antibodies under native conditions using fast protein liquid chromatography (FPLC; Amersham Pharmacia).

Immunohistochemistry on different tissue cryostat sections was performed as described by Castellani et al.(22 1994) and 4-18% gradient SDS-PAGE was carried out according to Carnemolla et al. (17 1989) under reducing and non-reducing conditions..

*Animals and cell lines*

Athymic-nude mice (8 week-old nude/nude CD1 females) were obtained from Harlan Italy (Correzzana, Milano, Italy), 129 (clone SvHsd) strain mice (8-10 weeks old, female) were obtained from Harlan UK (Oxon, England). Mouse embryonal teratocarcinoma cells (F9), human melanoma derived cells (SK-MEL-28) and mouse myeloma cells (SP2/0) were purchased from American Type Culture Collection (Rockville, MD). To induce tumors, nude mice were subcutaneously injected with $16 \times 10^6$ SK-MEL-28 cells, and 129 strain mice with $3 \times 10^6$ F9 cells. The tumor volume was determined with the following formula: $(d)^2 \times D \times 0,52$, where d and D are, respectively, the short and long dimensions (cm) of the tumor, measured with a caliper. Housing, treatments and sacrifice of animals were carried out according to national legislation (Italian law no. 116 of 27 January, 1992) regarding the protection of animals used for scientific purposes.

*Radioiodination of recombinant antibodies*

Radioiodination of proteins was achieved following the Chizzonite indirect method (36 Riske et al.,1991) using IODOGEN Pre-coated Iodination tubes (Pierce) to activate $Na^{125}I$ (NEN Life Science Products, Boston, MA) according to manufacturer's recommendations. In the reported experiments, 1.0 mCi of $Na^{125}I$ was used for 0.5mg of protein. The radiolabeled molecules were separated from free $^{125}I$ using PD10 (Amersham Pharmacia) columns pre-treated with 0.25% BSA and equilibrated in PBS. The radioactivity of the samples was established using a Crystal $\gamma$-counter (Packard Instruments, Milano, Italy). The immunoreactivity assay of the radiolabeled protein was performed on a 200$\mu$l ED-B Sepharose column saturated with 0.25% BSA in PBS. A known amount of radioiodinated antibody, in 200$\gamma$l of 0.25% BSA in PBS, was applied on top and allowed to enter the column. The column was then rinsed with 1.5 ml of 0.25% BSA in PBS to remove non-specifically bound antibodies. Finally, the immunoreactive bound material was eluted using 1.5 ml of 0.1M TEA, ph11. The radioactivity of unbound and bound material was counted and the percentage of immunoreactive antibodies was calculated. Immunoreactivity was always higher than 90%.

To further analyze the radioiodinated antibodies a known amount of radiolabeled protein in 200$\mu$l was loaded onto the Superdex 200 column. The retention volume of the different proteins did not vary after radioiodination. For the three radioiodinated L19 antibody formats and their negative controls, the radioactivity recovery from the Superdex 200 column was 100% (Figure 3A, 3B and 3C).

*Biodistribution experiments*

To block non-specific accumulation of $^{125}$ Iodine in the stomach and concentration in thyroid, 30 minutes before injection of the radiolabeled antibodies mice orally received 20 mg of sodium perchlorate (Carlo Erba, Italy) in water. This procedure was repeated at 24h intervals for the duration of biodistribution experiments. Tumor-bearing mice were injected in the tail vein with 0,1 nmoles of the different radiolabeled antibodies (corresponding to 6 $\mu$g for scFvs, 8 $\mu$g for SIPs and 18 $\mu$g for IgGs) in 100$\mu$l of saline. Three animals were sacrificed per time point, the different organs including tumor were excised, weighed, counted in a $\gamma$-counter and then fixed with 5% formaldehyde in PBS, pH 7.4, to be processed for microautoradiographies, performed according to Tarli et al. (23 1999).

The blood was sampled also for plasma preparation to determine the stability of the radiolabeled molecules in the blood stream using the already described immunoreactivity test and the gel filtration analysis. In both cases 200 $\mu$l of plasma were used. The radioactive content of the different organs was expressed as percentage of injected dose per gram (%ID/g).

The blood clearance parameters of the radioiodinated antibodies was fitted with a least squares minimization procedure, using the MacIntosh Program Kaleidagraph (Synergy Software, Reading PA, USA) and the equation:

$$X (t) = A \exp (-(alpha \ \ t)) + B \exp (-(beta \ t)$$

where X (t) is the %ID/g of radiolabeled antibody at time t. This equation describes a bi-exponential blood clearance profile, in which the amplitude of the alpha phase is defined as A x 100 / (A + B) and the amplitude of the beta elimination phase is defined as B x 100 / (A + B). Alpha and beta are rate parameters related to the half-lives of the corresponding blood clearance phases. T1/2 (alpha phase) = ln2/alpha = 0.692.../alpha T1/2 (beta phase) = ln2/alpha = 0.692... / alpha. X(0) was assumed to be equal to 40%, corresponding to a blood volume of 2.5 ml in each mouse.

*RESULTS*

Antibody preparation

Using the variable regions of L19 (13 Pini et al., 1998) different antibody formats (scFv, mini-immunoglobulin and complete human IgG1) and their performance *in vivo* in targeting tumoral vasculature.

Figure 1 shows the constructs used to express the different, L19 antibody formats. Similar constructs were prepared using the variable regions of the scFv specific for a non-relevant antigen (D1.3; 7 McCafferty; 26 Neri et al., 1997).

To obtain SIPs and IgG1, SP2/0 murine myeloma cells were transfected with the constructs shown in Figure 1 and stable transfectomas were selected using G418. The best producers were determined by ELISA and these clones were expanded for antibody purification. The purification of all three L19 antibody formats was based on immunoaffinity chromatography using recombinant ED-B conjugated to Sepharose. The yields were of about 8 mg/l for scFv(L19), 10mg/l for L19-SIP, 3 mg/l for L19-IgG1. For the control proteins were used scFv(D1.3) specific for hen-egg lysozyme, and, using the variable regions of scFv D1.3, D1.3-SIP was constructed. These two antibodies were purified on hen-egg lysozyme conjugated to Sepharose. The yields were of 8 and 5 mg/l, respectively. As control for L19-IgG1 we used commercially available human IgG1/κ (Sigma).

SDS-PAGE analysis of the three purified L19 formats was performed, under both reducing and non-reducing conditions. For scFv(L19), the apparent mass was, as expected, about 28 kDa under both reducing and non-reducing conditions (not shown). The L19-SIP showed a molecular mass of nearly 80 kDa under non-reducing conditions, and had a mass of about 40 kDa under reducing conditions. The results demonstrated that more than 95% of the native molecule exists as a covalently-linked dimer. L19-IgG1 showed, as expected, a main band of about 180 kDa under non-reducing conditions, while, under reducing conditions, it showed two bands corresponding to the heavy chain of about 55 kDa and the light chain of about 28 kDa. Elution profiles of the three L19 antibody formats analyzed by size exclusion chromatography (Superdex 200) were obtained. In all three cases a single peak with a normal distribution, and representing more than 98%, was detected. Using a standard calibration curve, the apparent molecular masses were 60 kDa for scFv(L19)$_2$, 80kDa for L19-SIP and 180kDa for L19-IgG1. In addition, molecular aggregates that are often present in recombinant protein preparations and that may invalidate the results obtained in *in vivo* studies were demonstrated to be absent. SDS-PAGE and size exclusion chromatography (Superdex 200) performed on the purified control proteins gave similar results.

Using these three different L19 antibody formats, immunohistochemical analyses were performed on cryostat sections of SK-MEL-28 human melanoma induced in nude mice, and of F9 murine teratocarcinoma induced in 129 strain mice. Optimal results were obtained at concentrations as low as 0.25-0.5 nM. All three purified L19 antibodies recognized identical structures.

*In vivo stability of the radiolabeled L19 antibody formats* For *in vivo* biodistribution studies, SK-MEL-28 human melanoma and F9 murine teratocarcinoma were used. SK-MEL-28 tumor has a relatively slow growth rate while, F9 tumor grows rapidly (Figure 2). Therefore, the use of SK-MEL-28 tumor enabled long-lasting experiments (up to 144h), while F9 tumor was induced for short biodistribution studies (up to 48h). All the biodistribution experiments were performed when the tumors were approximately 0.1-0.3 cm$^3$. For comparison of the various antibody formats, equimolar amounts (0.1 nmol) in 100μl of sterile saline were injected. Before injection, the radioiodinated compounds were filtered 0.22 μm and the immunoreactivity and gel filtration profile were checked (see Materials and Methods). Immunoreactivity of the radiolabeled proteins was always more than 90%.

Figure 3 A-C reports the profiles of the gel filtration analysis (Superdex 200) of the radioiodinated L19 antibody formats.

Blood samples were taken from treated animals at the different time intervals from injection and the radioactivity present in plasma was analyzed for immunoreactivity and by gel filtration chromatography. Gel filtration profiles showed a single major peak, having the molecular mass of the injected protein, for all three L19 antibody formats. Only the profile of the scFv revealed a second peak having a higher molecular mass, suggesting formation of aggregates (Figure 3 D-F). Furthermore, the formation of large molecular mass aggregates not eluting from the Superdex 200 column, was observed for scFv(L19)2. In fact, while the recovery from the Superdex 200 column was 90-100% of the applied radioactivity for both L19-SIP and L19-IgG, the yield of the loaded radioactivity of scFv(L19)2 was about 55%. The retained radioactivity was recovered only after washing the chromatography column with 0.5M NaOH, demonstrating that large aggregates were blocked on the column filter (Table 1).

Table 1 also reports the results of the immunoreactivity test performed on plasma (see Materials and Methods). Over the time of the experiments, L19-SIP and L19-IgG1 maintained the same immunoreactivity in plasma as the starting reagents. On the contrary, already 3 hours after injection the immunoreactivity of scFv(L19)2 in plasma was reduced to less than 40%.

*Comparative biodistribution experiments*

Tables 2 a, b, c and Figure 4 report the results obtained in the biodistribution experiments with the radiolabeled L19 antibodies in SK-MEL-28 tumor bearing mice.

Tables 2 a,b,c show, at different times from i.v. injection of the radiolabeled antibodies, the average ($\pm$SD) of the %ID/g of tissues and organs, including tumors.

In Figure 4 are depicted the variations of the %ID/g of the different antibody formats in tumor (A) and blood (B) at the different times of the experiments, as well as the ratios (C) between the %ID/g in tumor and blood. All three L19 antibody formats selectively accumulated in the tumor and the ratio of the %ID/g of tumor and other organs are reported in Table 3.

As demonstrated by microautoradiography, the antibodies accumulate only on the tumor vasculature, whereas no specific accumulation on the vasculature of normal organs was seen. By contrast, no specific accumulation of the radioiodinated control molecules in either tumors or normal tissues was found (Tables 2 a, b, c).

All three L19 antibody formats showed a clearance that was mediated mainly by the kidney, as determined by counting the urine samples. As expected, clearance rate was faster for scFv(L19)2 and slower for the complete L19-IgG1. Fitting of the curve with a biexponential function yielded the half-live values reported in Table 4.

Figure 5 depicts the variations in the %ID/g ($\pm$SD) of tumor and blood obtained with the radioiodinated scFv (L19)2 and L19-SIP using the F9 teratocarcinoma tumor model. Due to the high angiogenic activity of F9 teratocarcinoma, accumulation of radioactive molecules in this tumor was 3 to 4 times higher, 3 and 6 h after i.v. injection than in SK-MEL-28 tumor and was persistently higher for the 48h duration of the experiment. As for SK-MEL-28 tumor, specific accumulation in tumor vasculature was confirmed by microautoradiography, while no specific tumor accumulation was seen after injection of the control molecules. In Table 5 are reported the %ID/g of L19(scFv) and L19SIP, at different times after i.v. injection, in F9 tumors and other organs.

*Synthesis of reduced L19-SIP*

To a solution of 375$\mu$g (5nmol) L19-SIP in 422$\mu$l PBS were added 50$\mu$l TCEP-solution (14.34mg TCEPxHCl/5ml aqueous $Na_2HPO_4$, 0.1M, pH = 7.4). The reaction mixture was gently shaken for 1h at 37˚C. Reduced L19-SIP was purified by gel-chromatography using a NAP-5 column (Amersham, Eluant: PBS). SDS-PAGE analysis of the isolated product proofed the quantitative transformation of L19-SIP to reduced L19-SIP.
Yield: 100.3$\mu$g/200$\mu$l PBS (26.7%).

*Synthesis of Tc-99m-L19-SIP*

3.0 mg disodium-L-tartrate were placed in a vial followed by addition of 100.3$\mu$g reduced L19-SIP in 200$\mu$l PBS and the solution was diluted with 100$\mu$l aqueous $Na_2HPO_4$-buffe (1M, pH = 10.5). 85$\mu$l Tc-99m generator eluate (24h) and 10$\mu$l $SnCl_2$-solution (5mg $SnCl_2$/1ml 0.1M HCl) were added. The reaction mixture was shaken for 0.5h at 37˚C. Tc-99m-labeled L19-SIP was purified by gel-chromatography using a NAP-5 column (Amersham, Eluant: PBS).

| Radiochemical yield: | 35.6%. |
| --- | --- |
| Radiochemical purity: | 90.2% (SDS-PAGE). |
| Specific activity: | 26.4MBq/nmol. |
| Immunoreactivity: | 91.4% |

*Synthesis of Tc-99m-MAG$_2$-L19-SIP Carboxy methyl- t- butyl disulfide*

A solution of 21.75ml (0.312mol) 1-mercapto-acetic acid, 43.5ml (0.312mol) triethylamine and 100g (0.312mol) N-(tert.-butylthio)-N,N'-di-BOC-hydrazine in 11 EtOH (abs.) was heated under reflux (N$_2$-atmosphere) for 60h. EtOH was evaporated under reduced pressure to a final volume of about 200ml. The residue was poured in 1.81 H$_2$O and the pH of the resulting suspension was adjusted to 7.14 using 5molar NaOH. Di-BOC-hydrazine was filtered off and the pH of the resulting solution was adjusted to 2.2 using half-concentrated HCl. Crude material was extracted from water 3x with 600 ml CH$_2$Cl$_2$. The combined organic layers were dried over MgSO$_4$ and the solvent was evaporated under reduced pressure yielding 41.1g (80%) as a yellow oil. The material was pure enough for further synthesis.

*N-(benzyloxycarbonyl-Gly)Gly t-butyl ester (Z-(N-Gly)Gly t-butyl ester*

A solution of 35.02g (114mmol) Z-Gly-OSuccinimide and 15g (114mmol) Gly-0-$^t$Bu in 1.41 CH$_2$Cl$_2$ was stirred under N$_2$-atmosphere at room temperature for 20 h. The organic layer was washed 3x with 250ml 1% aqueous citric acid, 2x with 200ml half-saturated aqueous NaHCO$_3$ and 1x with 200ml water. The organic layer was dried over anhydrous MgSO$_4$. Evaporation of CH$_2$Cl$_2$ under reduced pressure yielded 36.5 g (99%) Z-Gly-Gly-O-$^t$Bu as a yellow oil. The crude material was pure enough for further synthesis.

*Gly-Gly t-butyl* ester

36.5 g (113 mmol) of Z-Gly-Gly-O$^t$Bu were dissolved in 11 THF followed by the addition of 3.65 g palladium on charcoal (10 %). The mixture was stirred under H$_2$ atmosphere (1atm) for 3h at room temperature. The suspension was purged with N$_2$, filtered (PTFE-filter: 0.45$\mu$m) and the filtrate was concentrated under reduced pressure yielding 20.3g (95%) Gly-Gly-O-$^t$Bu as a yellow oil. The crude material was pure enough for further synthesis.

*Carboxy methyl-t-butyl disulfide glycyl glycine t-butylester*

A solution of 23.85g (115.6mmol) DCC in 430ml CH$_2$Cl$_2$ was dropwise added to a solution of 21.76 g (115.6 mmol) Gly-Gly-O-$^t$Bu, 20.84 g (115.6 mmol) Carboxy methyl-t-butyl disulfide and 13.3 g (115.6 mmol) NHS in 1 1 CH$_2$Cl$_2$. The resulting suspension was stirred over night under N$_2$-atmosphere at room temperature. After filtration the resulting solution was washed 3x with 400ml half-saturated aqueous NaHCO$_3$ and 1x with 400 ml water. The dried organic layer (MgSO$_4$) was evaporated under reduced pressure. The crude product was purified by chromatography on silica gel using a solvent gradient ranging from CH$_2$Cl$_2$/MeOH 99:1 to CH$_2$Cl$_2$/MeOH 98.5:1.5. 26.1g (64 %) were isolated as a yellow oil.

*Mercaptoacetyl glycyl glycine*

26.32g (75.09mmol) Carboxy methyl-t-butyl disulfide glycyl glycine t-butyl ester were dissolved in 233ml TFA under N$_2$-atmosphere. The resulting solution was stirred for 20min at room temperature. TFA was evaporated under reduced pressure (5-10 x 10$^{-2}$mbar) and the resulting oil was dried under stirring for additional 2h (5-10 x 10$^{-2}$mbar). After addition of 250ml Et$_2$O a white powder precipitated and the suspension was stirred for 3h. The material was filtered off and resuspended in 100ml Et$_2$O. The resulting suspension was stirred over night, the product was filtered off and the material was dried at room temperature under reduced pressure yielding 20.46g (92.5%) as a white powder.

*Mercaptoacetyl glycyl glycine NHS ester*

Mercaptoacetyl glycyl glycine (1g, 3.4 mmol) and N-hydroxysuccinimide (391 mg, 3.4 mmol) are combined in a dry round bottom flask and dissolved in anhydrous DMF (4 ml). DCC (700 mg, 3.4 mmol) in anhydrous dioxane (2 ml) was added while stirring the reaction mixture. Within 15 min a precipitate (DCU) begins to form. After 1 h the precipitate is removed by vacuum filtration. The precipitate was washed with cold dioxane. The dioxane was removed from the filtrate. The product was precipitated from the remaining DMF solution by adding diethylether. The product was isolated by filtration, washed with cold diethylether, and dried in a vacuum desiccator overnight. Yield: 1.33 (99 %).

*Synthesis of Tc-99m-MAG$_2$-ε-HN (Lys)-L19-SIP*

200 $\mu$g (2.66 nmol) non-reduced L19-SIP in 111 $\mu$l PBS were diluted with 300 $\mu$l of sodium borate buffer (0.1M, pH 8.5) and dialyzed 2 x 1 h with 200ml of phosphate buffer (0.1M, pH 8.5) employing a Slide-A-Lyzer 10,000 MWCO (Pierce Inc., Rockford, IL, U.S.A.). 50 $\mu$l of mercaptoacetyl glycyl glycine NHS ester solution (0.50 mg dissolved in 500

μl of phosphate buffer, 0.1M, pH 8.5) were added and the reaction mixture was heated for 3 h at 37°C. The reaction mixture was dialyzed 2 x 1 h and 1 x 17 h (over night) with 200 ml of phosphate buffer (0.1M, pH 8.5) each, employing the Slide-A-Lyzer 10,000 MWCO (Pierce Inc., Rockford, IL, U.S.A.). 3.0 mg disodium-L-tartrate were added to the vial followed by addition of. 90 μl Tc-99m generator eluate (eluated daily) and 25μl SnCl$_2$-solution (5mg SnCl$_2$/1ml 0.1M HCl) were added. The reaction mixture was shaken for 0.5h at 37°C. Tc-99m-labeled L19-SIP was purified by gel-chromatography using a NAP-5 column (Amersham, Eluent: PBS).

| | |
|---|---|
| Radiochemical yield: | 55.1 %. |
| Radiochemical purity: | 94.5 % (SDS-PAGE). |
| Specific activity: | 15.2 MBq/nmol. |
| *Immunoreactivity:* | *81.1 %Synthesis of Re-188-L19-SIP* |

3.0 mg disodium-L-tartrate were placed in a vial followed by addition of 150μg reduced L19-SIP-SH in 310μl PBS and the solution was diluted with 100μl aqueous Na$_2$HPO$_4$-buffe (1M, pH = 10.5). 100μl Re-188 generator eluate and 50μl SnCl$_2$-solution (5mg SnCl$_2$/1ml 0.1M HCl) were added. The reaction mixture was shaken for 1.5h at 37°C. Re-188-labeled L19-SIP was purified by gel-chromatography using a NAP-5 column (Amersham, Eluent: PBS).

| | |
|---|---|
| Radiochemical yield: | 34.8 %. |
| Radiochemical purity: | 97.2 % (SDS-PAGE). |
| Specific activity: | 13.5 MBq/nmol. |
| Immunoreactivity: | 91.7 % |

*Synthesis of reduced L19-SIP for specific conjugation of EDTA, CDTA, TETA, DTPA, TTHA, HBED, DOTA, NOTA, DO3A, and a like type chelators to the Cysteine-SH group*

50μl TCEP-solution (14.34mg TCEPxHCl/5ml aqueous Na$_2$HPO$_4$, 0.1M, pH = 7.4) were added to a solution of 375μg (5 nmol) L19-SIP in 422μl PBS. The reaction mixture was gently shaken for 1h at 37°C. Reduced L19-SIP was purified by gel-chromatography using a NAP-5 column (Amersham, Eluent: sodium acetate buffer, 0.1M, pH 5.0). SDS-PAGE analysis of the isolated product proofed the quantitative transformation of L19-SIP into reduced L19-SIP. Yield: 105.7μg/200μl (28.2%).

*Synthesis of In-111-MX-DTPA-Maleimide-S(Cys)-L19-SIP-R (R = reduced)*

105 μg (2.8 nmol) reduced L19-SIP in 200 μl of sodium acetate buffer (0.1M, pH 5) were reacted with 50μl of dissolved 1,4,7-triaza-2-(N-maleimido ethylene p-amino)benzyl-1,7-bis(carboxymethyl)-4-carboxymethyl 6-methyl heptane (0,25mg DTPA-Maleimide in 500μl sodium acetate buffer 0.1M pH 5) for 3 h at 37°C. The reaction mixture was dialyzed 2 x 1 h with 200ml of sodium acetate buffer (0.1M, pH 6) employing a Slide-A-Lyzer 10,000 MWCO (Pierce Inc., Rockford, IL, U.S.A.).
80 μl [In-111]InCl$_3$ solution (HCl, 1N, 40 MBq, Amersham Inc.) were added and the reaction mixture was heated at 37°C for 30 min.
In-111 labeled DTPA-Maleimide-S(Cys)-L19-SIP was purified by gel-chromatography using a NAP-5 column (Amersham, Eluent: PBS).

| | |
|---|---|
| Radiochemical yield: | 51.6 %. |
| Radiochemical purity: | 97.2 % (SDS-PAGE). |
| Specific activity: | 7.9 MBq/nmol. |
| Immunoreactivity: | 88.5 % |

*Synthesis of MX-DTPA-Maleimide (1,4,7-triaza-2-(N-maleimido ethylene p-amino)benzyl-1,7-bis(carboxymethyl)-4-carboxymethyl 6-methyl heptane)*

512 mg (1 mmol) of {[3-(4-Amino-phenyl)-2-(bis-carboxymethyl-amino)-propyl]-[2-(bis-carboxymethyl-amino)-propyl]-amino}-acetic acid (Macrocyclics Inc. Dallas, TX, U.S.A.) and 707 mg (7 mmol) triethylamine were dissolved in 3 ml dry DMF. 400 mg (1,5 mmol) of 3-(2,5-Dioxo-2,5-dihydro-pyrrol-1-yl)-propionic acid 2,5-dioxo-pyrrolidin-1-yl ester (Aldrich) in 1 ml dry DMF were added drop-wisely. The solution was stirred for 5 h at 50° C. 30 ml of diethylether were added slowly. The reaction mixture was stirred for further 30 min. The precipitate was collected by filtering. The crude product was purified by RP-HPLC (acetonitrile- : water- : trifluoracetic acid / 3 : 96,9 : 0,1 → 99,9 : 0 : 0,1). Yield: 61% (405 mg, 0,61 mmol). MS-ESI: 664 = M$^+$ +1.

*Synthesis of In-111-MX-DTPA-ε-HN (Lys)-L19-SIP*

200 μg (2.66 nmol) non-reduced L19-SIP in 111 μl PBS were diluted with 300 μl of sodium borate buffer (0.1M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 μl of 1,4,7-triaza-2-(p-isothiocyanato)benzyl-1,7-bis(carboxymethyl)-4-carboxymethyl-6-methyl heptane (MX-DTPA) solution (0.33 mg MX-DTPA dissolved in 500 μl of sodium borate buffer, 0.1M, pH 8.5) were added and the reaction mixture was heated for 3 h at 37˚C. The reaction mixture was dialyzed 2 x 1 h and 1 x 17 h (over night) with 200 ml of sodium acetate buffer (0.1M, pH 6.0) each, employing the Slide-A-Lyzer 10,000 MWCO (Pierce Inc., Rockford, IL, U.S.A.).

80 μl [In-111]InCl$_3$ solution (HCl, 1N, 40 MBq, Amersham Inc.) were added and the reaction mixture was heated at 37˚C for 30 min. In-111 labeled MX-DTPA-ε-HN(Lys)-L19-SIP was purified by gel-chromatography using a NAP-5 column (Amersham, Eluent: PBS).

| | |
|---|---|
| Radiochemical yield: | 72.4 %. |
| Radiochemical purity: | 80.3 % (SDS-PAGE). |
| Specific activity: | 8.8 MBq/nmol. |
| Immunoreactivity: | 77.5 % |

*Synthesis of In-111 -DOTA-C-Benzyl-p-NCS -ε-HN (Lys)-L19-SIP*

200 μg (2.66 nmol) non-reduced L19-SIP in 108 μl PBS were diluted with 300 μl of sodium borate buffer (0.1M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 μl of 1,4,7,10-tetraaza-2-(p-isothiocyanato)benzyl cyclododecane-1,4,7,10-tetraacetic acid (benzyl-p-SCN-DOTA, Macrocyclics Inc., Dallas TX, U.S.A.) solution (1.5 mg benzyl-p-SCN-DOTA dissolved in 5 ml of sodium borate buffer, 0.1M, pH 8.5) were added to the solution and the reaction mixture was heated for 3 h at 37˚C. The reaction mixture was dialyzed 2 x 1 h and 1 x 17 h (over night) with 200 ml of sodium acetate buffer (0.1M, pH 6.0) each, employing the Slide-A-Lyzer 10,000 MWCO (Pierce Inc., Rockford, IL, U.S.A.).

80 μl [In-111]InCl$_3$ solution (HCl, 1N, 40 MBq, Amersham Inc.) were added and the reaction mixture was heated at 37˚C for 30 min. In-111 labeled DOTA-C-Benzyl-p-NCS-ε-HN(Lys)-L19-SIP was purified by gel-chromatography using a NAP-5 column (Amersham, Eluent: PBS).

| | |
|---|---|
| Radiochemical yield: | 70.8 %. |
| Radiochemical purity: | 92.1 % (SDS-PAGE). |
| Specific activity: | 10.1 MBq/nmol. |
| Immunoreactivity: | 75.1 % |

*Synthesis of Y-88-MX-DTPA-ε-HN (Lys)-L19-SIP*

200 μg (2.66 nmol) non-reduced L19-SIP in 110 μl PBS were diluted with 300 μl of sodium borate buffer (0.1M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 μl of MX-DTPA solution (0.33 mg MX-DTPA dissolved in 500 μl of sodium borate buffer, 0.1M, pH 8.5) were added and the reaction mixture was heated for 3 h at 37˚C. The reaction mixture was dialyzed 2 x 1 h and 1 x 17. h (over night) with 200 ml of sodium acetate buffer (0.1M, pH 6.0) each, employing the Slide-A-Lyzer 10,000 MWCO (Pierce Inc., Rockford, IL, U.S.A.).

100 μl [Y-88]YCl$_3$ solution (HCl, 1N, 75 MBq, Oak Ridge National Lab.) were added and the reaction mixture was heated at 37˚C for 30 min. Y-88 labeled MX-DTPA-ε-HN(Lys)-L19-SIP was purified by gel-chromatography using a NAP-5 column (Amersham, Eluent: PBS).

| | |
|---|---|
| Radiochemical yield: | 68.1 %. |
| Radiochemical purity: | 91.5 % (SDS-PAGE). |
| Specific activity: | 11.4 MBq/nmol. |
| Immunoreactivity: | 70.5% |

*Synthesis of Lu-177 -DOTA-C-Benzyl-p-NCS -ε-HN (Lys)-L19-SIP*

200 μg (2.66 nmol) non-reduced L19-SIP in 120 μl PBS were dissolved with 300 μl of sodium borate buffer (0.1M, pH 8.5) and dialyzed 2 x 1 h with 200ml of sodium borate buffer (0.1M, pH 8.5) employing a Slide-A-Lyzer 10,000 MWCO (Pierce Inc., Rockford, IL, U.S.A.). 50 μl of benzyl-p-SCN-DOTA solution (1.5 mg dissolved in 5 ml of sodium borate buffer, 0.1M, pH 8.5) were added and the reaction mixture was heated for 3 h at 37˚C. The reaction mixture was dialyzed 2 x 1 h and 1 x 17 h (over night) with 200 ml of sodium acetate buffer (0.1M, pH 6.0) each, employing the Slide-A-Lyzer 10,000 MWCO (Pierce Inc., Rockford, IL, U.S.A.).

200 μl [Lu-177]LuCl$_3$ solution (HCl, 1N, 80 MBq, NRH-Petten, Netherlands) were added and the reaction mixture was heated at 37˚C for 30 min. Lu-177 labeled DOTA-C-Benzyl-p-NCS-ε-HN(Lys)-L19-SIP was purified by gel-chromatography using a NAP-5 column (Amersham, Eluent: PBS).

| | |
|---|---|
| Radiochemical yield: | 72.2 %. |
| Radiochemical purity: | 94.9 % (SDS-PAGE). |
| Specific activity: | 18.3 MBq/nmol. |
| Immunoreactivity: | 73.4 % |

*Organ distribution and excretion of In-111-MX-DTPA-L19-SIP after a single i.v. injection into tumor-bearing nude mice*

The labeled peptides of the invention were injected intravenously in a dose of about 37 kBq into F9 (teratocarcinoma)-bearing animals (body weight about 25 g). The radioactivity concentration in various organs, and the radioactivity in the excreta, was measured using a γ counter at various times after administration of the substance.

The biodistribution of In-111-MX-DTPA-L19-SIP in F9 (teratocarcinoma)-bearing nude mice (mean ± SD, n=3) is shown in Table 6.

*Organ distribution and excretion of Tc-99m-L19-SIP after a single i.v. injection into tumor-bearing nude mice*

Labeled peptides were injected intravenously in a dose of about 56 kBq into F9 (teratocarcinoma)-bearing animals (bodyweight about 25 g). The radioactivity concentration in various organs, and the radioactivity in the excreta was measured using a γ counter at various times after administration of the substance. In addition, the tumor to blood ratio was found at various times on the basis of the concentration of the peptide in tumor and blood.

The biodistribution of Tc-99m-L19-SIP in F9 (teratocarcinoma)-bearing nude mice (mean ± SD, n=3) is shown in Table 7.

The tumor to blood ratio of Tc-99m-L19-SIP in F9 (teratocarcinoma)-bearing nude mice (mean ± SD, n=3) is shown in Table 8.

Radiolabeled peptides proved to possess favorable properties in animal experiments. For example, Tc-99m-L19-SIP and In-111-MX-DTPA-ε-HN(Lys)-L19-SIP displayed high tumor accumulation of 17.2 (Tc-99m ) or 12.9 (In-111) % injected dose per gram (ID/g) at 1 hour post injection (p.i.). Significant tumor retention of 9.4 (Tc-99m) or 13.0 (In-111) % ID/g at 24 hours p.i. was observed. Thus, tumor uptake is significantly higher compared to other known In-111 or Tc-99m labeled antibody fragments (e.g. Kobayashi et al., J. Nuc. Med., Vol. 41(4), pp. 755 - 762, 2000; Verhaar et al., J. Nuc. Med., Vol. 37(5), pp. 868 - 872, 1996). The compound's blood clearance lead to tumor/blood ratios of 13: 1 and 6:1 respectively, at 24 h p.i.

Most remarkably In-111-MX-DTPA-ε-HN(Lys)-L19-SIP displayed significantly lower kidney uptake and retention (22.5 % ID/g) than other highly retained In-111 labeled recombinant antibody fragment (120 % ID/g) described e.g. by Kobayashi et al. at 24 h p.i. Kidney retention is a very common problem and usually hampers the use of lanthanide labeled compounds in radiotherapy.

The experimental results demonstrate the excellent potential of the radioimmunoconjugates described herein for diagnostic and therapeutic applications, preferably applied to the patient by parenteral administration.

*DISCUSSION*

The observation that cytotoxic anticancer drugs localize more efficiently in normal tissues than in tumors (37 Bosslet et al., 1998) prompted a wave of studies investigating the possibility of selective drug delivery to tumors. The effective targeting of tumors, however, has two main requisites: 1) a target in the tumor that is specific, abundant, stable and readily available for ligand molecules coming from the bloodstream, and 2) a ligand molecule with suitable pharmakokinetic properties that is easily diffusible from the bloodstream to the tumor and with a high affinity for the target to ensure its efficient and selective accumulation in the tumor.

Due to its distinctive features the tumor microenvironment is a possible pan-tumoral target. In fact, tumor progression induces (and subsequently needs) significant modifications in tumor micro-environment components, particularly those of the extracellular matrix (ECM). The molecules making up the ECM of solid tumors differ both quantitatively and qualitatively from those of the normal ECM. Moreover, many of these tumor ECM components are shared by all solid tumors, accounting for general properties and functions such as cell invasion (both normal cells into tumor tissues and cancer cells into normal tissues) and angiogenesis. Of the numerous molecules constituting the modified tumor ECM, the present inventors have focused attention on a FN isoform containing the ED-B domain (B-FN).

B-FN is widely expressed in the ECM of all solid tumors thus far tested and is constantly associated with angiogenic processes (22 Castellani et al., 1994), but is otherwise undetectable in normal adult tissues (17 Carnemolla et al., 1989). Targeted delivery of therapeutic agents to the subendothelial ECM overcomes problems associated with interstitial hypertension of solid tumors (38 Jain et al. 1988; 39 Jain, 1997; 40 Jain RK, 1999).

L19 (13 Pini et al. 1998; 25 Viti, Canc.Res., 23 Tarli, et al., 1999), an scFv with a high affinity (Kd=5.4x10$^{-11}$M) for the ED-B domain of FN, selectively and efficiently accumulates *in vivo* around tumor neo-vasculature and is able to selectively transport and concentrate in the tumor mass any one of a number of therapeutic molecules to which it is conjugated (28 Birchler et al., 1999; 29 Nilsson, et al., 2001; 30 Halin et al.2002; 31 Carnemolla et al., 2002). The ability of L19 to selectively target tumors has also been demonstrated in patients using scintigraphic techniques.

The present specification reports on labelling of small immunoprotein (SIP) with radioisotopes, use of the radiolabelled SIP, and on tumor vascular targeting performance and pharmacokinetics of three different L19 human antibody formats: the scFv, the mini-immunoglobulin/small immunoprotein and complete human IgG1.

The SIP molecule was obtained by fusion of the scFv(L19) to the εCH4 domain of the secretory isoform S$_2$ of human IgE. The εCH4 is the domain that allows dimerization of IgE molecules and the S$_2$ isoform contains a cysteine at the COOH terminal that covalently stabilizes the dimer through an interchain disulphide bond (35 Batista et al., 1996). The IgE binding sites for FCεRI reside in the CH3 domain (41 Turner and Kinet, 1999; 42 Vangelista et al., 1999; 43 Garman et al., 2000), so scFv fused to εCH4 domain in accordance with embodiments of the present invention does not activate any signalling leading to hypersensitivity reactions.

The performance of these three formats in two different tumor models in mouse has been studied: in murine F9 teratocarcinoma and human SK-MEL-28 melanoma. The first is a rapidly growing tumor that, once implanted, kills the animals in about two weeks. SK-MEL-28 tumor, on the other hand, presents a biphasic growth curve, with an early, fast, growth phase followed by a second, slower, phase. It has previously been shown that the amount of ED-B in F9 teratocarcinoma remains stable during tumor growth (23 Tarli, et al., 1999); by contrast, ED-B accumulates in SK-MEL-28 melanoma proportionally to the ability of the tumor to grow (23 Tarli et al., 1999), with abundant ED-B being found in the first phase and a lesser amount in the second. The use of SK-MEL-28 melanoma tumor allowed long-term biodistribution studies without dramatic variations of tumoral mass (Figure 2) that could give rise to misinterpretation of results.

Comparative studies of the three L19 antibody formats in terms of stability *in vivo* showed that L19-SIP and L19-IgG1 maintained, for the duration of experiments (144h), the same immunoreactivity and molecular mass in plasma as before injection. By contrast, scFv(L19) rapidly lost its immunoreactivity in plasma and generated aggregates that were too large to enter the gel filtration chromatography column. Such aggregation of the scFv is very likely responsible for the ratio between %ID/g of tumor and lung, since aggregates could accumulate in the microvasculature of the lung (Table 3). For all three formats, the blood clearance is mediated mainly via the kidney, showing a biphasic curve with an α and a β phase, reported in Table 4, which is inversely proportional to molecular size.

The accumulation of the different antibody formats in the tumors studied was a consequence of the clearance rate and *in vivo* stability of the molecules. Using the scFv, the maximum percent injected dose per gram (%ID/g) was observed 3h after injection of the radiolabeled antibody and then rapidly decreased. Using the SIP, the %ID/g in tumors was 2-5 times higher than that of the scFv, reaching a maximum 4-6 hours after injection. This pattern was observed in both F9 and SK-MEL-28 tumors. By contrast, the accumulation of IgG1 in tumors rose constantly during the experiments. However, due to its slow clearance, the tumor-blood ratio of the %ID/g after 144 hours was only about 3, compared to a ratio of 10 for the scFv and 70 for the SIP after the same period of time (Figure 4).

The same distinctive properties of *in vivo* stability, clearance and tumor targeting performance shown by the three antibody formats studied here may be exploited for different diagnostic and/or therapeutic purposes, depending on the clinical needs and disease. For instance, radiolabeled antibodies showing good tumor-organ and tumor-blood ratios soon after injection are necessary for *in vivo* diagnostic immunoscintigraphy, mainly because short half-life isotopes are used in such analysis.

Different approaches are possible using antibody as a vehicle for therapeutic agents: delivery of substances that display their therapeutic effects after reaching their targets (e.g., photosensitisers activated only on the targets), for which the absolute amount delivered to the tumor is relevant; delivery of substances that exert their therapeutic and toxic effects even before reaching the target (e.g., the β-emitter Yttrium-90), for which particular attention must be given to the ratio of the area under the curves of tumor and blood accumulation as a function of time, in order to minimize the systemic toxicity and to maximize the anti-tumor therapeutic effect.

L19-SIP, for instance, seems to offer the best compromise of molecular stability, clearance rate and tumor accumulation. Similar fusion proteins composed of scFv antibody fragments bound to a dimerizing domain have already been described (44 Hu et al, 1996; 33 Li et al., 1997), but in both cases the human $\gamma$1CH3 was used as the dimerizing domain. The usage of the human $\varepsilon_{s2}$CH4 domain provides an easy way of getting a covalent stabilization of the dimer. In addition, the disulphide bridge formed by the C-terminal cysteine residues can be easily reduced in mild enough conditions to preserve the overall structure of the molecule, thus providing a readily accessible reactive group for radiolabelling or chemical conjugation. This feature seems particularly promising in the view of the clinical potential.

L19-IgG1 gathers abundantly in tumors, and even though this accumulation is offset by a slow blood clearance rate, the three step procedure to remove circulating antibodies may be used to allow its use not only for therapeutic purposes but also for diagnostic immunoscintigraphy (45 Magnani et al. 2000) .

*REFERENCES*

1. Shawler et al. J.Immunol., 135: 1530-1535, 1985
2. Miller et al. Blood, 62: 988-995, 1983.
3. Hakimi et al. J.Immunol., 147: 1352-1359,1991.
4. Stephens et al. Immunology, 85: 668-674, 1995.
5. Riechmann et al. Nature, 332: 323-327, 1988.
6. Junghans et al. Cancer Res., 50: 1495-1502, 1990.
7. McCafferty et al. Nature, 348: 552-554, 1990.
8. Lowman et al. Biochemistry, 30: 10832-10838, 1991
9. Nilsonn et al. Advanced Drug Delivery Reviews, 43: 165-196, 2000.
10. Winter et al. Annu.Rev.Immunol., 12: 433-455, 1994.
11. Reichert. Nature Biotech., 19: 819-822, 2001.
12. Huls et al. Nature Biotech., 17: 276-281, 1999,
13. Pini et al. J Biol Chem, 273: 21769-21776, 1998.
14. Neri and Zardi. Advanced Drug Delivery Reviews, 31: 43-52, 1998.
15. Bissel and Radisky. Nature Reviews - Cancer., 1: 46-54, 2001
16. Balza et al. FEBS Lett., 228: 42-44, 1988
17. Carnemolla et al. J. Cell. Biol., 108: 1139-1148, 1989
18. Borsi et al. FEBS Lett., 261: 175-178, 1990
19. Borsi et al. J. Biol.Chem., 270: 6243-6245, 1995.
20. Zardi et al. EMBO J., 6: 2337-2342, 1987.
21. ffrench-Constant et al. J.Cell Biol., 109: 903-914, 1989.
22. Castellani et al. Int J Cancer, 59: 612-618, 1994
23. Tarli et al. Blood, 94:192-198, 1999.
24. Carnemolla et al. Int J Cancer, 68: 397-405, 1996
25. Viti et al. Cancer Res, 59: 347-353, 1999.
26. Neri et al. Nature Biotechnol, 15: 1271-1275, 1997.
27. Demartis et al. Eur J Nucl Med, 28: 4534-4539, 2001.
28. Birchler et al. Nat Biotechnol, 17: 984-988, 1999
29. Nilsson et al. Cancer Res., 61: 711-716, 2001.
30. Halin et al. Nature Biotechnol. In the press, 2002.
31. Carnemolla et al. Blood , 99 :, 2002
32. Wu et al. Proc. Nat. Acad. Sci. U.S.A., 97: 8495-8500, 2000.
33. Li et al. Protein Engineering, 10: 731-736, 1997
34. Pini et al. J. Immunol. Methods, 206: 171-183, 1997.
35. Batista et al. J. Exp. Med., 184: 2197-205, 1996.
36. Riske et al. J.Biol. Chem., 266: 11245-11251, 1991.
37. Bosslet et al. Cancer Res., 58:1195-1201, 1998.
38. Jain and Baxter. Cancer Res., 48: 7022-7032, 1988.
39. Jain. Vascular and interstitial physiology of tumors. Role in cancer detection and treatment. In: R.Bicknell, C.E. Lewis and N. Ferrara (eds). Tumour Angiogenesis, pp. 45-59. New York: Oxford University Press, 1997.
40. Jain. Annu. Rev. Biomed. Eng., 1: 241-263, 1999.
41. Turner and Kinet. Nature, 402 Suppl., B24-B30, 1999.

42. Vangelista et al. Jour. Clin. Invest., 103:1571-1578, 1999.
43. Garman et al Nature, 406: 259-266, 2000.
44. Hu et al. Cancer Res., 56: 3055-3061, 1996.
45. Magnani et al. Br. J. Cancer, 82: 616-620, 2000.

**Table 1. Immunoreactivity (I[*]) and radiactivity recovery (R) from Superdex 200 of the radiolabeled antibodies, at different times after i.v. injection**

| Time (h) | 3 | | 6 | | 24 | | 48 | | 72 | | 144 | |
|---|---|---|---|---|---|---|---|---|---|---|---|---|
| | I | R | I | R | I | R | I | R | I | R | I | R |
| L19 (scFv) | 36 | 54 | 32 | 58 | 27 | nd | 14 | nd | 9 | nd | 4 | nd |
| L19SIP | 100 | 100 | 100 | 96 | 100 | 94 | 95 | 96 | 100 | nd | 95 | nd |
| L19IgG 1 | 100 | 100 | 100 | 100 | 95 | 100 | 100 | 100 | 100 | 100 | 95 | 100 |

-Immunoreactivity (%) and radioactivity recovery (%) from Superdex200 were determined in plasma as described in Materials and Methods.
- *To normalize, the results of the immunoreactivity test are referred to the percentage values of the immunoreactivity before i.v. injection.
- nd : not determined

**Table 2 a. Biodistribution experiments of radiolabeled L19 and D1.3 antibody fragments in SK-MEL-28 tumor-bearing mice**

| L19(scFv) | 3h | 6h | 24h | 48h | 72h | 144h |
|---|---|---|---|---|---|---|
| TUMOR | 2,47 ± 0,65 | 2,01 ± 0,72 | 1,62 ± 0,43 | 0,95 ± 0,14 | 0,68 ± 0,04 | 0,32 ± 0,14 |
| Blood | 1,45 ± 0,58 | 0,54 ± 0,12 | 0,10 ± 0,03 | 0,04 ± 0,01 | 0,03 ± 0,02 | 0,03 ± 0,01 |
| Liver | 0,48 ± 0,20 | 0,18 ± 0,05 | 0,04 ± 0,01 | 0,02 ± 0,00 | 0,02 ± 0,01 | 0,02 ± 0,00 |
| Spleen | 0,67 ± 0,28 | 0,27 ± 0,04 | 0,07 ± 0,02 | 0,03 ± 0,00 | 0,02 ± 0,01 | 0,02 ± 0,00 |
| Kidney | 4,36 ± 0,32 | 1,67 ± 0,08 | 0,16 ± 0,01 | 0,06 ± 0,01 | 0,04 ± 0,02 | 0,03 ± 0,00 |
| Intestine | 0,77 ± 0,21 | 0,57 ± 0,05 | 0,24 ± 0,06 | 0,17 ± 0,04 | 0,12 ± 0,05 | 0,09 ± 0,01 |
| Heart | 0,77 ± 0,20 | 0,31 ± 0,07 | 0,07 ± 0,02 | 0,02 ± 0,00 | 0,02 ± 0,01 | 0,02 ± 0,00 |
| Lung | 2,86 ± 0,34 | 1,50 ± 0,67 | 1,07 ± 0,42 | 0,73 ± 0,39 | 0,55 ± 0,11 | 0,51 ± 0,22 |
| D1.3(scFv) | 3h | 6h | 24h | 48h | 72h | 144h |
| TUMOR | 1,03 ± 0,74 | 0,87 ± 0,42 | 0,15 ± 0,10 | 0,07 ± 0,02 | nd | nd |
| Blood | 1,52 ± 0,86 | 0,81 ± 0,13 | 0,02 ± 0,00 | 0,01 ± 0,00 | nd | nd |
| Liver | 1,19 ± 0,65 | 0,66 ± 0,26 | 0,14 ± 0,04 | 0,03 ± 0,08 | nd | nd |
| Spleen | 1,05 ± 0,88 | 0,42 ± 0,33 | 0,07 ± 0,02 | 0,05 ± 0,01 | nd | nd |
| Kidney | 3,01 ± 2,48 | 1,83 ± 0,76 | 0,48 ± 0,01 | 0,18 ± 0,05 | nd | nd |
| Intestine | 0,56 ± 0,54 | 0,56 ± 0,13 | 0,17 ± 0,03 | 0,02 ± 0,01 | nd | nd |
| Heart | 0,86 ± 0,54 | 0,55 ± 0,84 | 0,02 ± 0.01 | 0,01 ± 0,00 | nd | nd |
| Lung | 1,28 ± 0,65 | 1,06 ± 0,88 | 0,04 ± 0,01 | 0,03 ± 0,01 | nd | nd |

The results are expressed as percent of antibody injected dose per gram of tissue (%ID/g)±SD nd: not determined

**Table 2 b. Biodistribution experiments of radiolabeled L19-SIP and D1.3-SIP in SK-MEL-28 tumor-bearing mice**

| L19 SIP | 3h | 6h | 24h | 48h | 72h | 144h |
|---|---|---|---|---|---|---|
| TUMOR | 5,23 ± 0,65 | 6,14 ± 2,23 | 4,20 ± 2,47 | 2,57 ± 0,31 | 2,33 ± 0,90 | 1,49 ± 0,65 |
| Blood | 9,82 ± 0,68 | 5,03 ± 0,52 | 1,39 ± 0,06 | 0,29 ± 0,04 | 0,08 ± 0,02 | 0,02 ± 0,01 |
| Liver | 2,65 ± 0,14 | 1,74 ± 0,31 | 0,50 ± 0,04 | 0,19 ± 0,01 | 0,10 ± 0,02 | 0,05 ± 0,01 |

(continued)

| L19 SIP 3h | | 6h | 24h | 48h | 72h | 144h |
|---|---|---|---|---|---|---|
| Spleen | 3,76 ± 0,36 | 2,43 ± 0,24 | 0,71 ± 0,05 | 0,26 ± 0,04 | 0,13 ± 0.01 | 0,17 ± 0,18 |
| Kidney | 7,33 ± 0,91 | 3,87 ± 0,21 | 1,09 ± 0,05 | 0,30 ± 0,04 | 0,14 ± 0,02 | 0,05 ± 0,01 |
| Intestine | 1,45 ± 0,24 | 1,44 ± 0,29 | 1,06 ± 0,43 | 0,56 ± 0,08 | 0,40 ± 0,08 | 0,18 ± 0,00 |
| Heart | 4,16 ± 0,30 | 2,15 ± 0,08 | 0,52 ± 0,05 | 0,13 ± 0,03 | 0,06 ± 0,01 | 0,02 ± 0,01 |
| Lung | 7,72 ± 0,60 | 5,41 ± 0,55 | 1,81 ± 0,40 | 0,59 ± 0,29 | 0,19 ± 0,03 | 0,05 ± 0,01 |
| **D1.3SIP** | **3h** | **6h** | **24h** | **48h** | **72h** | **144h** |
| TUMOR | 3,80 ± 0,30 | 1,65 ± 0,12 | 0,70 ± 0,00 | 0,26 ± 0,01 | 0,07 ± 0,01 | 0,04 ± 0,03 |
| Blood | 10,40 ± 0,81 | 4,45 ± 0,14 | 1,21 ± 0,01 | 0,32 ± 0,00 | 0,08 ± 0,01 | 0,06 ± 0,02 |
| Liver | 4,05 ± 0,98 | 2,73 ± 0,33 | 1,43 ± 0,07 | 0,51 ± 0,21 | 0,15 ± 0,08 | 0,02 ± 0,01 |
| Spleen | 3,31 ± 0,66 | 1,76 ± 0,50 | 0,82 ± 0,12 | 0,46 ± 0,20 | 0,15 ± 0,05 | 0,04 ± 0,02 |
| Kidney | 8,41 ± 0,49 | 4,64 ± 0,06 | 1,47 ± 0,05 | 0,36 ± 0,03 | 0,16 ± 0,03 | 0,06 ± 0,01 |
| Intestine | 2,03 ± 0,55 | 1,06 ± 0,20 | 1,02 ± 0,06 | 0,14 ± 0,03 | 0,08 ± 0,02 | 0,12 ± 0,04 |
| Heart | 3,28 ± 0,20 | 1,81 ± 0,02 | 0,29 ± 0,01 | 0,06 ± 0,00 | 0,05 ± 0,01 | 0,04 ± 0,01 |
| Lung | 6,16 ± 0,28 | 4,52 ± 0,07 | 1,16 ± 0,05 | 0,09 ± 0,00 | 0,06 ± 0,01 | 0,05 ± 0.01 |

The results are expressed as percent of antibody injected dose per gram of tissue (%ID/g)±SD nd: not determined

**Table 2 c. Biodistribution experiments of radiolabeled L19IgG1 and hIgG1k in SK-MEL-28 tumor-bearing mice**

| L19 IgG1 | 3h | 6h | 24h | 48h | 72h | 144h |
|---|---|---|---|---|---|---|
| TUMOR | 4,46 ± 0,08 | 5,39 ± 1,01 | 6,70 ± 2,10 | 7,80 ± 2,51 | 8,90 ± 2,52 | 11,22 ± 3,19 |
| Blood | 16,04 ± 0,81 | 12,02 ± 1,65 | 8,31 ± 1,77 | 5,12 ± 1,42 | 5,02 ± 3,81 | 4,87 ± 0,26 |
| Liver | 6,03 ± 0,37 | 6,77 ± 0,53 | 2,41 ± 0,35 | 1,45 ± 0,41 | 1,26 ± 0,71 | 1,09 ± 0,16 |
| Spleen | 6,63 ± 1,34 | 6,37 ± 1,37 | 2,51 ± 0,47 | 2,01 ± 0,32 | 1,80 ± 1,02 | 1,51 ± 0,29 |
| Kidney | 6,47 ± 0,39 | 5,12 ± 0,47 | 3,07 ± 0,35 | 1,73 ± 0,63 | 1,54 ± 1,14 | 1,12 ± 0,44 |
| Intestine | 1,60 ± 0,39 | 1,35 ± 0,65 | 1,43 ± 0,19 | 1,13 ± 0,32 | 1,13 ± 0,98 | 0,97 ± 0,47 |
| Heart | 5,63 ± 0,67 | 4,77 ± 0,52 | 2,87 ± 0,45 | 1,48 ± 0,51 | 1,32 ± 1,09 | 0,92 ± 0,37 |
| Lung | 6,55 ± 0,65 | 5,15 ± 0,62 | 4,16 ± 0,66 | 2,28 ± 0,80 | 1,98 ± 1,60 | 1,42 ± 0,45 |
| **hIgG1k** | **3h** | **6h** | **24h** | **48h** | **72h** | **144h** |
| TUMOR | nd | 3,28 ± 0,38 | 4,00 ± 0,22 | 2,78 ± 0,20 | nd | 2,32 ± 0,26 |
| Blood | nd | 10,12 ± 0,35 | 7,87 ± 0,25 | 6,24 ± 0,34 | nd | 5,41 ± 0,51 |
| Liver | nd | 4,02 ± 0,09 | 2,06 ± 0,10 | 1,90 ± 0,24 | nd | 1,28 ± 0,03 |
| Spleen | nd | 4,47 ± 0,28 | 1,82 ± 0,01 | 1,42 ± 0,19 | nd | 1,24 ± 0,03 |
| Kidney | nd | 5,40 ± 0,19 | 2,56 ± 0,06 | 2,08 ± 0,22 | nd | 1,30 ± 0,15 |
| Intestine | nd | 0,72 ± 0,07 | 0,46 ± 0,05 | 0,36 ± 0,03 | nd | 0,31 ± 0,01 |
| Heart | nd | 3,80 ± 0,15 | 2,52 ± 0,21 | 0,99 ± 0,18 | nd | 1,48 ± 0,13 |
| Lung | nd | 4,82 ± 0,92 | 3,64 ± 0,08 | 1,75 ± 0,32 | nd | 1,09 ± 0,13 |

The results are expressed as percent of antibody injected dose per gram of tissue (%ID/g)±SD nd: not determined

**Table 3. Tumor-organ ratios of the % ID/g of the radiolabeled L19 antibody formats in SK-MEL-28 tumor-bearing mice.**

| | L19(ScFv) | | | | | | L19SIP | | | | | | L19IgG1 | | | | | |
|---|---|---|---|---|---|---|---|---|---|---|---|---|---|---|---|---|---|---|
| Time(h) | 3 | 6 | 24 | 48 | 72 | 144 | 3 | 6 | 24 | 48 | 72 | 144 | 3 | 6 | 24 | 48 | 72 | 144 |
| **TUMOR** | 1 | 1 | 1 | 1 | 1 | 1 | 1 | 1 | 1 | 1 | 1 | 1 | 1 | 1 | 1 | 1 | 1 | 1 |
| Blood | 1,7 | 3,7 | 16,2 | 23,7 | 22,7 | 10,7 | 0,5 | 1,2 | 3,0 | 8,9 | 29,1 | 74,5 | 0,3 | 0,4 | 0,8 | 1,5 | 1,8 | 2,3 |
| Liver | 5,1 | 11,1 | 40,5 | 47,5 | 34,0 | 16,0 | 2,0 | 3,5 | 8,4 | 13,5 | 23,3 | 29,8 | 0.7 | 0,8 | 2,8 | 5,4 | 7,1 | 6,3 |
| Spleen | 3,7 | 7,4 | 23,1 | 31,6 | 34,0 | 16,0 | 1,4 | 2,5 | 5,9 | 10,0 | 17,9 | 8,8 | 0,7 | 0,6 | 2,7 | 3,9 | 4,9 | 7,4 |
| Kidney | 0,6 | 1,2 | 10,1 | 15,8 | 17,0 | 10,7 | 0,7 | 1,6 | 3,8 | 8,6 | 16,6 | 29,8 | 0,7 | 1,0 | 2,2 | 4,5 | 5,8 | 5,3 |
| Intestine | 3,2 | 3,5 | 6,7 | 5,6 | 5,7 | 3,6 | 3,6 | 4,3 | 4,0 | 4,6 | 5,8 | 8,3 | 2,8 | 4,0 | 4,7 | 6,9 | 7,9 | 7,1 |
| Heart | 3,2 | 6,5 | 23,1 | 47,5 | 34,0 | 16,0 | 1,3 | 2,9 | 8,1 | 20,0 | 38,8 | 74,5 | 0,8 | 1,1 | 2,3 | 5,3 | 6,7 | 5,8 |
| Lung | 0,9 | 1,3 | 1,5 | 1,3 | 1,2 | 0,6 | 0,7 | 1,1 | 2,3 | 4,3 | 12,3 | 29,8 | 0,7 | 1,0 | 1,6 | 3,4 | 4,5 | 3,7 |

**Table 4. Kinetic parameters for blood clearance of the three L19 antibody formats**

| | α | | β | |
|---|---|---|---|---|
| | (%)[a] | $t_{1/2}$(h) | (%)[a] | $t_{1/2}$(h) |
| **L19(scFv)** | 96,7 | 0,53 | 3,3 | 8,00 |
| **L19-SIP** | 83,7 | 1,06 | 16,3 | 10,66 |
| **L19-IgG1** | 76,9 | 1,48 | 23,1 | 106,7 |

[a] Relative magnitude of the two half-life components

**Table 5. Biodistribution experiments of radiolabeled L19(scFv) and L19SIP in F9 tumor-bearing mice**

| L19(scFv) | 3h | 6h | 24h | 48h |
|---|---|---|---|---|
| **TUMOR** | **10,46** ± 1,75 | **8,15** ± 2,63 | **3,18** ± 0,83 | **2,83** ± 0,71 |
| **Blood** | **2,05** ± 0,38 | **1,88** ± 1,14 | **0,17** ± 0,01 | **0,06** ± 0,02 |
| **Liver** | **1,62** ± 1,67 | **0,73** ± 0,51 | **0,07** ± 0,01 | **0,04** ± 0,02 |
| **Spleen** | **1,53** ± 0,36 | **0,90** ± 0,54 | **0,11** ± 0,01 | **0,05** ± 0,01 |
| **Kidney** | **12,70** ± 0,73 | **4,37** ± 0,98 | **0,24** ± 0,03 | **0,18** ± 0,08 |
| **Intestine** | **0,68** ± 0,15 | **0,95** ± 0,23 | **0,24** ± 0,01 | **0,17** ± 0,06 |
| **Heart** | **1,35** ± 0,21 | **0,81** ± 0,38 | **0,08** ± 0,02 | **0,04** ± 0,01 |
| **Lung** | **2,88** ± 0,29 | **2,06** ± 0,69 | **0,38** ± 0.60 | **0,33** ± 0,05 |
| L19SIP | 3h | 6h | 24h | 48h |
| **TUMOR** | **17,46** ± 1,93 | **16,65** ± 2,59 | **15,32** ± 2,17 | **12,00** ± 1,91 |
| **Blood** | **13,51** ± 0,57 | **9,62** ± 1,18 | **1,73** ± 0,02 | **1,14** ± 0,20 |
| **Liver** | **2,81** ± 0,37 | **2,39** ± 0,13 | **0,54** ± 0,14 | **0,32** ± 0,00 |
| **Spleen** | **3,42** ± 0,26 | **2,66** ± 0,27 | **0,61** ± 0,09 | **0,37** ± 0,01 |
| **Kidney** | **9,18** ±0,76 | **5,85** ± 0,50 | **1,16** ± 0,05 | **0,76** ± 0,06 |
| **Intestine** | **0,95** ± 0,03 | **1,36** ± 0,21 | **0,83** ± 0,11 | **1,04** ± 0,14 |
| **Heart** | **4,64** ± 0,24 | **3,67** ± 0,46 | **0,67** ± 0,06 | **0,46** ± 0,07 |
| **Lung** | **5,61** ± 0,01 | **5,93** ± 0,57 | **1,66** ± 0,19 | **0,91** ± 0,08 |

The results are expressed as percent of antibody injected dose per gram of tissue (%ID/g)±SD nd: not determined

Table 6

| | % of dose / g of tissue | | |
|---|---|---|---|
| | 1h p.i. | 3h p.i. | 24h p.i. |
| Spleen | 5.05 ± 1.04 | 4.27 ± 0.27 | 4.86 ± 1.77 |
| Liver | 10.80 ± 1.52 | 10.57 ± 1.44 | 10.68 ± 1.51 |
| Kidney | 14.30 ± 1.45 | 16.71 ± 2.42 | 22.48 ± 6.79 |
| Lung | 9.94 ± 1.72 | 6.15 ± 0.80 | 3.03 ± 0.95 |
| Stomach without contents | 1.10 ± 0.13 | 1.62 ± 0.19 | 1.66 ± 0.24 |
| Intestine with contents | 1.67 ± 0.14 | 2.65 ± 0.30 | 2.64 ± 1.40 |
| Tumour | 12.93 ± 2.76 | 10.18 ± 2.28 | 12.96 ± 3.13 |
| Blood | 17.10 ± 1.49 | 9.08 ± 0.96 | 1.98 ± 0.47 |

Table 7

| | % of dose / g of tissue | | |
| --- | --- | --- | --- |
| | 1h p.i. | 3h p.i. | 24h p.i. |
| Spleen | 6.92 ± 1.3 | 5.37 ± 0.23 | 2.06 ± 0.48 |
| Liver | 14.65 ± 0.81 | 12.43 ± 0.37 | 4.62 ± 0.52 |
| Kidney | 22.07 ± 1.87 | 15.99 ± 1.10 | 5.92 ± 1.18 |
| Lung | 10.06 ± 1.67 | 5.33 ± 0.49 | 1.32 ± 0.25 |
| Stomach without contents | 2.18 ± 0.39 | 2.12 ± 0.09 | 1.15 ± 0.08 |
| Intestine with contents | 3.03 ± 0.25 | 3.62 ± 0.58 | 1.20 ± 0.12 |
| Tumour | 17.20 ± 7.49 | 18.79 ± 5.35 | 9.42 ± 3.84 |
| Blood | 16.53 ± 2.04 | 7.42 ± 0.21 | 0.73 ± 0.14 |

Table 8

| | 1h p.i. | 3h p.i. | 24h p.i. |
| --- | --- | --- | --- |
| Tumour to blood ratio | 1.01 ± 0.33 | 2.54 ± 0.74 | 12.81 ± 4.03 |

Statements of invention

1. A specific binding member that binds human ED-B, wherein the specific binding member is labelled with an isotope selected from the group consisting of $^{76}$Br, $^{77}$Br, $^{123}$I, $^{124}$I, $^{131}$I and $^{211}$At and comprises an antigen-binding site that comprises an antibody VH domain and an antibody VL domain, wherein the antibody VH domain is selected from the group consisting of the L19 VH domain, and a VH domain comprising a VH CDR1, a VH CDR2 and a VH CDR3, wherein the VH CDR3 is the L19 VH CDR3 of SEQ ID NO. 3, the VH CDR1 is optionally L19 VH CDR1 of SEQ ID NO. 1, and the VH CDR2 is optionally L19 VH CDR2 of SEQ ID NO. 2; and wherein the antibody VL domain is optionally selected from the group consisting of the L19 VL domain, and a VL domain comprising a VL CDR1, a VL CDR2 and a VL CDR3, wherein the VL CDR3 is the L19 VL CDR3 of SEQ ID NO. 6, the VL CDR1 is optionally L19 VL CDR1 of SEQ ID NO. 4, and the VL CDR2 is optionally L19 VL CDR2 of SEQ ID NO. 5; the L19 VH domain and L19 VL domain sequences being disclosed in Pini et al. (1998) J. Biol. Chem. 273: 21769-21776; wherein the specific binding member comprises a mini-immunoglobulin comprising said antibody VH domain and antibody VL domain fused to $\varepsilon_{s2}$-CH4 and dimerized or comprises a whole IgG1 antibody molecule.

2. A specific binding member according to 1 comprising an antibody VH domain comprising the VH CDR's with the amino acid sequences of SEQ ID NO. 1, SEQ ID NO. 2 and SEQ ID NO. 3, which specific binding member competes for binding to ED-B with an ED-B-binding domain of an antibody comprising the L19 VH domain and the L19 VL domain.

3. A specific binding member according to 1 or 2 comprising the L19 VH domain.

4. A specific binding member according to 3 comprising the L19 VL domain.

5. A specific binding member according to any one of the preceding which is a mini-immunoglobulin comprising $\varepsilon_{S2}$-CH4.

6. A specific binding member according to 5 wherein the antibody VH domain and antibody VL domain are within an scFv antibody molecule fused to $\varepsilon_{S2}$-CH4.

7. A specific binding member according to 6 wherein the scFv antibody molecule is fused to $\varepsilon_{S2}$-CH4 via a linker peptide.

8. A specific binding member according to 7 wherein the linker peptide has the amino acid sequence GGSG (SEQ ID NO. 7).

9. A specific binding member according to any one of 1 to 4 that comprises a whole IgG1 antibody molecule.

10. A specific binding member according to any of 1 to 9 wherein the isotope is $^{131}$I.

11. A method of producing a specific binding member according to any one of 1 to 10, the method comprising labelling a specific binding member with an isotope selected from the group consisting of $^{76}$Br, $^{77}$Br, $^{123}$I, $^{124}$I, $^{131}$I and $^{211}$At.

12. A method according to 11, wherein the labelling comprises oxidising a halogenide selected from the group consisting of $^{76}$Br$^-$, $^{77}$Br$^-$, $^{123}$I$^-$, $^{124}$I$^-$, $^{131}$I$^-$ and $^{211}$At$^-$ in the presence of the specific binding member.

13. A method according to 11, wherein the labelling comprises conjugating an activated bi-functional halogen carrier containing a radioiosotope selected from the group consisting of $^{76}$Br, $^{77}$Br, $^{123}$I, $^{129}$I, $^{131}$I and $^{211}$At to a lysine or a cysteine residue or to the N terminus of the specific binding member.

14. A method according to any one of 11 to 13, wherein the method comprises expressing nucleic acid encoding the specific binding member prior to the labelling.

15. A method according to 14, comprising culturing host cells under conditions for production of the specific binding member.

16. A method according to any one of 11 to 15, further comprising isolating and/or purifying the specific binding member.

17. A method according to any one of 11 to 16 further comprising formulating the specific binding member into a composition including at least one additional component.

18. A method according to any one of 11 to 17 further comprising binding the specific binding member to ED-B or a fragment of ED-B.

19. A method comprising binding a specific binding member that binds ED-B according to any one of 1 to 10 to ED-B or a fragment of ED-B.

20. A method according to 18 or 19 wherein said binding takes place *in vitro.*

21. A method according to any one of 18 to 20 comprising determining the amount of binding of specific binding member to ED-B or a fragment of ED-B.

22. A composition comprising a specific binding member according to any one of 1 to 10, for use in a method of treatment of the human or animal body by therapy.

23. A composition according to 22 for use in a method of treatment of a lesion of pathological angionesis.

24. A composition according to 22 for use in a method of treatment of a tumor.

25. A composition according to any one of 1 to 10 for use in a method of diagnosis.

26. Use of a specific binding member according to any one of 1 to 10 in the manufacture of a medicament for treating a lesion of pathological angiogenesis.

27. Use of a specific binding member according to any one of 1 to 10 in the manufacture of a medicament for treating a tumor.

28. Use of a specific binding member according to any one of 1 to 10 in the manufacture of a diagnostic reagent.

**Claims**

1.  A specific binding member that binds human ED-B, wherein the specific binding member is labelled with an isotope selected from the group consisting of $^{131}$I, $^{76}$Br, $^{77}$Br, $^{123}$I, $^{124}$I and $^{211}$At and comprises an antigen-binding site that comprises an antibody VH domain and an antibody VL domain, wherein the antibody VH domain comprises a VH CDR1, a VH CDR2 and a VH CDR3, wherein the VH CDR3 is the L19 VH CDR3 of SEQ ID NO. 3, the VH CDR1 is the L19 VH CDR1 of SEQ ID NO. 1, and the VH CDR2 is the L19 VH CDR2 of SEQ ID NO. 2; and wherein the antibody VL domain is optionally a VL domain comprising a VL CDR1, a VL CDR2 and a VL CDR3, wherein the VL CDR3 is the L19 VL CDR3 of SEQ ID NO. 6, the VL CDR1 is the L19 VL CDR1 of SEQ ID NO. 4, and the VL CDR2 is the L19 VL CDR2 of SEQ ID NO. 5; wherein the specific binding member comprises a whole IgG1 antibody molecule.

2.  A specific binding member according to claim 1, which specific binding member competes for binding to ED-B with an ED-B-binding domain of an antibody comprising the L19 VH domain and the L19 VL domain.

3. A specific binding member according to claim 1 or claim 2 comprising the L19 VH domain and optionally the L19 VL domain.

4. A specific binding member according to any of claims 1 to 3 wherein the isotope is $^{131}$I.

5. A method of producing a specific binding member according to any one of claims 1 to 4, the method comprising labelling a specific binding member with an isotope selected from the group consisting of $^{131}$I, $^{76}$Br, $^{77}$Br, $^{123}$I, $^{124}$I and $^{211}$At.

6. A method according to claim 5, wherein the labelling comprises:

   (i) oxidising a halogenide selected from the group consisting of $^{131}$I⁻, $^{76}$Br⁻, $^{77}$Br⁻, $^{123}$I⁻, $^{124}$I⁻ and $^{211}$At⁻ in the presence of the specific binding member; or
   (ii) conjugating an activated bi-functional halogen carrier containing a radioiosotope selected from the group consisting of $^{131}$I, $^{76}$Br, $^{77}$Br, $^{123}$I, $^{124}$I and $^{211}$At to a lysine or a cysteine residue or to the N terminus of the specific binding member.

7. A method according to claim 5 or 6, wherein the method comprises expressing nucleic acid encoding the specific binding member prior to the labelling.

8. A method according to claim 7, comprising culturing host cells under conditions for production of the specific binding member.

9. A method according to any one of claims 5 to 8, further comprising isolating and/or purifying the specific binding member.

10. A method according to any one of claims 5 to 9 further comprising formulating the specific binding member into a composition including at least one additional component.

11. A method according to any one of claims 5 to 10 further comprising binding the specific binding member to ED-B or a fragment of ED-B *in vitro.*

12. A method comprising binding a specific binding member that binds ED-B according to any one of claims 1 to 4 to ED-B or a fragment of ED-B, wherein said binding takes place *in vitro.*

13. A method according to claim 11 or 12 comprising determining the amount of binding of specific binding member to ED-B or a fragment of ED-B.

14. A composition comprising a specific binding member according to any one of claims 1 to 4, for use in a method of treatment of the human or animal body by therapy.

15. A composition according to claim 14 for use in a method of treatment of a lesion of pathological angionesis.

16. A composition according to claim 14 for use in a method of treatment of a tumor.

17. A composition according to any one of claims 1 to 4 for use in a method of diagnosis.

## Figure 1A

## Figure 1B

L19scFv/pDN322

## Figure 1C

L19SIP/pCDNA3

## Figure 1D

L19HIgG1/pcDNA3

L19kL/pCMV2Δ

Figure 2

Figure 3A

Figure 3D

Figure 3B

Figure 3E

Figure 3C

Figure 3F

## Figure 4A

SKMEL-28 TUMOR

## Figure 4B

BLOOD

## Figure 4C

T-B RATIO

## Figure 5A

F9 tumor — L19SIP / L19(scFv)2; %ID/g vs time (h)

## Figure 5B

blood — L19SIP / L19(scFv)2; %ID/g vs time (h)

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0162298 A
- GB 9701412 W
- EP 9903210 W
- EP 0102062 W
- WO 0100382 W
- EP 184187 A
- GB 2188638 A
- EP 239400 A
- EP 0120694 A
- EP 0125023 A

### Non-patent literature cited in the description

- **Pini et al.** *J. Biol. Chem.,* 1998, vol. 273, 21769-21776
- **Kabat, E.A. et al.** Sequences of Proteins of Immunological Interest. US Department of Health and Human Services, 1991
- **Carrasquillo J.A. et al.** *J Nucl Med,* 1999, vol. 40, 268-276
- **Ledermann J.A. et al.** *Int J. Cancer,* 1991, vol. 47, 659-664
- **Bagshawe K.D. et al.** *Antibody, Immunoconjugates and Radiopharmaceuticals,* 1991, vol. 4, 915-922
- **Plückthun, A.** *Bio/Technology,* 1991, vol. 9, 545-551
- **Ref, M.E.** *Curr. Opinion Biotech.,* 1993, vol. 4, 573-576
- **Trill J.J. et al.** *Curr. Opinion Biotech,* 1995, vol. 6, 553-560
- **Sambrook et al.** Molecular Cloning: a Laboratory Manual. Cold Spring Harbor Laboratory Press, 2001
- Current Protocols in Molecular Biology. John Wiley & Sons, 1992
- **Kobayashi et al.** *J. Nuc. Med.,* 2000, vol. 41 (4), 755-762
- **Verhaar et al.** *J. Nuc. Med.,* 1996, vol. 37 (5), 868-872
- **Viti et al.** Canc.Res. 1999, vol. 25, 23
- **Shawler et al.** *J.Immunol.,* 1985, vol. 135, 1530-1535
- **Miller et al.** *Blood,* 1983, vol. 62, 988-995
- **Hakimi et al.** *J.Immunol.,* 1991, vol. 147, 1352-1359
- **Stephens et al.** *Immunology,* 1995, vol. 85, 668-674
- **Riechmann et al.** *Nature,* 1988, vol. 332, 323-327
- **Junghans et al.** *Cancer Res.,* 1990, vol. 50, 1495-1502
- **McCafferty et al.** *Nature,* 1990, vol. 348, 552-554
- **Lowman et al.** *Biochemistry,* 1991, vol. 30, 10832-10838
- **Nilsonn et al.** *Advanced Drug Delivery Reviews,* 2000, vol. 43, 165-196
- **Winter et al.** *Annu.Rev.Immunol.,* 1994, vol. 12, 433-455
- **Reichert.** *Nature Biotech.,* 2001, vol. 19, 819-822
- **Huls et al.** *Nature Biotech.,* 1999, vol. 17, 276-281
- **Pini et al.** *J Biol Chem,* 1998, vol. 273, 21769-21776
- **Neri ; Zardi.** *Advanced Drug Delivery Reviews,* 1998, vol. 31, 43-52
- **Bissel ; Radisky.** *Nature Reviews - Cancer.,* 2001, vol. 1, 46-54
- **Balza et al.** *FEBS Lett.,* 1988, vol. 228, 42-44
- **Carnemolla et al.** *J. Cell. Biol.,* 1989, vol. 108, 1139-1148
- **Borsi et al.** *FEBS Lett.,* 1990, vol. 261, 175-178
- **Borsi et al.** *J. Biol.Chem.,* 1995, vol. 270, 6243-6245
- **Zardi et al.** *EMBO J.,* 1987, vol. 6, 2337-2342
- **ffrench-Constant et al.** *J.Cell Biol.,* 1989, vol. 109, 903-914
- **Castellani et al.** *Int J Cancer,* 1994, vol. 59, 612-618
- **Tarli et al.** *Blood,* 1999, vol. 94, 192-198
- **Carnemolla et al.** *Int J Cancer,* 1996, vol. 68, 397-405
- **Viti et al.** *Cancer Res,* 1999, vol. 59, 347-353
- **Neri et al.** *Nature Biotechnol,* 1997, vol. 15, 1271-1275
- **Demartis et al.** *Eur J Nucl Med,* 2001, vol. 28, 4534-4539
- **Birchler et al.** *Nat Biotechnol,* 1999, vol. 17, 984-988

- **Nilsson et al.** *Cancer Res.,* 2001, vol. 61, 711-716
- **Halin et al.** *Nature Biotechnol.,* 2002
- **Carnemolla et al.** *Blood,* 2002, vol. 99
- **Wu et al.** *Proc. Nat. Acad. Sci. U.S.A.,* 2000, vol. 97, 8495-8500
- **Li et al.** *Protein Engineering,* 1997, vol. 10, 731-736
- **Pini et al.** *J. Immunol. Methods,* 1997, vol. 206, 171-183
- **Batista et al.** *J. Exp. Med.,* 1996, vol. 184, 2197-205
- **Riske et al.** *J.Biol. Chem.,* 1991, vol. 266, 11245-11251
- **Bosslet et al.** *Cancer Res.,* 1998, vol. 58, 1195-1201
- **Jain ; Baxter.** *Cancer Res.,* 1988, vol. 48, 7022-7032
- Vascular and interstitial physiology of tumors. Role in cancer detection and treatment. **Jain.** Tumour Angiogenesis. Oxford University Press, 1997, 45-59
- **Jain.** *Annu. Rev. Biomed. Eng.,* 1999, vol. 1, 241-263
- **Turner ; Kinet.** *Nature,* 1999, vol. 402, B24-B30
- **Vangelista et al.** *Jour. Clin. Invest.,* 1999, vol. 103, 1571-1578
- **Garman et al.** *Nature,* 2000, vol. 406, 259-266
- **Hu et al.** *Cancer Res.,* 1996, vol. 56, 3055-3061
- **Magnani et al.** *Br. J. Cancer,* 2000, vol. 82, 616-620